(19)

Europäisches
Patentamt
European
Patent Office
Office européen
des brevets

(11) **EP 2 537 025 B1**

(12) **EUROPEAN PATENT SPECIFICATION**

(45) Date of publication and mention
of the grant of the patent:
**27.04.2016 Bulletin 2016/17**

(51) Int Cl.:
**G01N 33/68** *(2006.01)*    **G01N 33/86** *(2006.01)*

(21) Application number: **11744189.9**

(86) International application number:
**PCT/AU2011/000164**

(22) Date of filing: **16.02.2011**

(87) International publication number:
**WO 2011/100792 (25.08.2011 Gazette 2011/34)**

(54) **PROTEIN BIOMARKERS FOR OBSTRUCTIVE AIRWAYS DISEASES**

PROTEINBIOMARKER FÜR OBSTRUKTIVE ATEMWEGSERKRANKUNGEN

BIOMARQUEURS PROTÉIQUES POUR MALADIES PULMONAIRES OBSTRUCTIVES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.02.2010 AU 2010900622**

(43) Date of publication of application:
**26.12.2012 Bulletin 2012/52**

(73) Proprietor: **Newcastle Innovation Limited
Callaghan, NSW 2308 (AU)**

(72) Inventors:
• **GIBSON, Peter
Merewether, New South Wales 2291 (AU)**
• **WOOD, Lisa
Merewether, New South Wales 2291 (AU)**
• **VERRILLS, Nikki
Rothbury, New South Wales 2320 (AU)**
• **SIM, Alistair
Callaghan, New South Wales 2308 (AU)**

(74) Representative: **Kalhammer, Georg et al
Lederer & Keller
Patentanwälte Partnerschaft mbB
Unsöldstrasse 2
80538 München (DE)**

(56) References cited:
**WO-A1-2008/109730    WO-A1-2009/114292
WO-A2-2006/105252    WO-A2-2007/084485
WO-A2-2007/095126    US-A1- 2007 141 585**

• **SANDFORD AJ ET AL: "Genetic risk factors for chronic obstructive pulmonary disease", EUROPEAN RESPIRATORY JOURNAL, vol. 10, 1997, pages 1380-1391, XP002698317, DOI: 10.1183/09031936.97.10061380**
• **BAKER CS ET AL: "Measurement of Ceruloplasmin in the Lungs of Patients with Acute Respiratory Distress Syndrome: Is Plasma or Local Production the Major Source?", CLINICAL INVESTIGATIONS, vol. 67, 2000, pages 533-538, XP002698318,**
• **VAN RENSEN ELIZABETH L J ET AL: "Assessment of microvascular leakage via sputum induction: The role of substance P and neurokinin A in patients with asthma", AMERICAN JOURNAL OF RESPIRATORY AND CRITICAL CARE MEDICINE, AMERICAN LUNG ASSOCIATION, NEW YORK, NY, US, vol. 165, no. 9, 1 May 2002 (2002-05-01), pages 1275-1279, XP008162672, ISSN: 1073-449X, DOI: 10.1164/RCCM.2110092**
• **ZAK-NEJMARK T ET AL: "HISTAMINE BINDING PROTEINS IDENTIFIED IN HEALTHY SUBJECTS AND ASTHMATIC PATIENTS", ARCHIVUM IMMUNOLOGIAE ET THERAPIAE EXPERIMENTALIS, BIRKHAEUSER VERLAG AG, CH, vol. 34, no. 5-6, 1 January 1986 (1986-01-01), pages 547-552, XP008162674, ISSN: 0004-069X**
• **SYROMYATNIKOVA N V ET AL: "[CLINICAL AND BIOCHEMICAL FINDINGS IN PATIENTS WITH NONSPECIFIC DISEASES OF THE RESPIRATORY ORGANS]", TERAPEVTICHESKII ARKHIV - THERAPEUTIC ARCHIVES, MOSCOW, RU, vol. 52, no. 7, 1 January 1980 (1980-01-01), pages 36-37, XP008162673, ISSN: 0040-3660**

**(Cont. next page)**

- LERU P. ET AL.: 'Study of fluticasone propionate efficacy in the treatment of patients with bronchial asthma not controlled by other inhaled corticosteroids.' REV. ROUM. MED. INT. vol. 36, no. 1-2, 1998, pages 105 - 111
- DATABASE DATABASE MEDLINE STN: 'Use of fluimucyl-antibiotic IT: antibiotic therapy advance for patients with acute attack of infectious chronic obstructive lung disease.' Database accession no. 2006264596 & LIKARS'KA SPRAVA / MINISTERSTVO OKHORONY ZDOROV'IA UKRAINY 2006, pages 65 - 81
- SCHOONBROOD D. F. M. ET AL.: 'Analysis of plasma-protein leakage and local secretion in sputum from patients with asthma and chronic obstructive pulmonary disease.' AMERICAN JOURNAL OF RESPIRATORY AND CRITICAL CARE MEDICINE vol. 150, no. 6, 1994, pages 1519 - 27
- SCHOONBROOD D. F. M. ET AL.: 'Plasma protein leakage and local secretion of proteins assessed in sputum in asthma and COPD: The effect of inhaled corticosteroids.' CLINICA CHIMICA ACTA vol. 240, 1995, pages 163 - 178
- ENGSTRöM G. ET AL.: 'Plasma markers of inflammation and incidence of hospitalisations for COPD: results from a population-based cohort study.' THORAX vol. 64, 2009, pages 211 - 5
- WONG W.S. F. ET AL. INT. ARCH. ALLERGY IMMUNOL. vol. 147, 2008, pages 179 - 189
- KIM K.-H. ET AL.: 'Proteomic analysis of human BALF; comparison of bronchial asthma with normal control.' MOLECULAR & CELLULAR PROTEOMICS vol. 5, no. 10, 2006, page S154.

## Description

### Field of the Invention

The present invention relates generally to the use of biomarkers for the diagnosis of obstructive airways diseases, in particular asthma and chronic obstructive pulmonary disease, based on differential protein expression profiles of such biomarkers. The invention also relates to methods for the diagnosis of obstructive airways diseases utilising such biomarkers.

### Background of the Invention

The obstructive airways diseases asthma and chronic obstructive pulmonary disease (COPD) are significant and increasing health problems throughout the world and despite continuing efforts there remains the need for improved approaches for the diagnosis of these diseases.

Both asthma and COPD are chronic inflammatory disorders of the airways. Whilst inflammation is a natural feature in the lungs of healthy individuals, for example to effect the removal of pathogens such as bacteria and viruses and pollutants which are present in the air, in the lungs of asthma and COPD sufferers an exaggerated response occurs in response to irritants (hyperresponsiveness). Increased airway inflammation follows exposure to inducers such as pollutants, allergens, viruses, exercise, or non-specific irritant inhalation. Increased inflammation leads to exacerbations characterized by shortness of breath, chest tightness, wheezing, coughing, and dyspnoea.

Asthma is the most widespread chronic health problem in the Western world and is increasing in prevalence around the world. In Australia alone, it affects over 2 million individuals. Worldwide bronchial asthma is the most common chronic disease in childhood with an overall prevalence between 5 and 20 percent across all ages. The pathogenesis is suggested to be complex and is centred on an aberrant immune response to inhalant allergens, most commonly house dust mite (HDM) allergens, that results in an inflammation of the airway wall together with an episodic constriction of the airways resulting in symptoms such as shortness of breath, wheezing, coughing, and life-threatening dyspnoea. COPD results from inhalation of pollutants, most often tobacco smoke but also a range of other airborne pollutants for example due to industrial and occupational exposure and urban air pollution. COPD can be regarded as a group of related diseases including chronic bronchitis and emphysema. COPD is a major and increasing health burden for the world. Indeed estimates suggest that there are more than 24 million sufferers in the United States alone.

Currently asthma is typically diagnosed by detection of episodic symptoms and by variable and reversible airflow obstruction, whereas COPD is diagnosed by incompletely reversible (fixed) airflow obstruction. The management of these diseases could be improved by improved diagnosis and recognition, and better understanding of their pathogenesis, leading to effective therapies. The need for improved diagnosis refers not only to the identification of individuals suffering from, or at risk of developing, an obstructive airways disease, but also the distinction between asthma and COPD. As potential treatment options for these diseases differ there is a clear need for diagnostic and prognostic indicators that effectively distinguish between COPD and asthma.

Asthma is also now recognised as a heterogeneous disease. This heterogeneity manifests itself at many different levels and is crucially important in patient care. Distinct asthma subtypes identified to date include eosinophilic asthma, paucigranulocytic asthma and neutrophilic asthma. Asthma subtypes differ in their inflammatory responses and pathways elicited upon exposure to allergens, and also in their clinical presentation. Current approaches to asthma diagnosis using symptoms and lung function mask this heterogeneity, resulting in inappropriate diagnosis. Furthermore, different asthma subtypes respond to different asthma therapies and inaccurate diagnosis can result in inappropriate treatment. For example, eosinophilic asthma is very sensitive to corticosteroids, whereas paucigranulocytic and neutrophilic asthma are not. Symptomatic paucigranulocytic asthma and neutrophilic asthma are treated by reducing corticosteroids, and using long acting bronchodilators and other antiinflammatory agents, such as macrolides.

As newer therapies for asthma focus on increasingly specific mechanistic pathways, identification of different forms of asthma will assume greater importance. This concept has been demonstrated in a randomised trial by Green et al. (2002, Lancet, 360:1715-1721), whereby basing asthma treatment on sputum eosinophils, a 50% reduction in asthma exacerbations was observed. This important effect is all the more significant since it was seen over and above current best practice using British Thoracic Society guidelines. In order to capitalise on this new knowledge of heterogeneity in asthma, measurements that are simple, repeatable, and have good performance characteristics are needed. There is significant potential for biomarkers of asthma phenotypes to impact on diagnosis, be used to detect phenotype and guide therapy.

New technologies for investigating human diseases now offer significant potential to address the need for improved diagnosis and better understanding of asthma and COPD. Proteomic analysis, utilizing high-resolution 2D-gel electrophoresis coupled with mass spectrometry, is a powerful means to identify differential protein expression between biological samples and thus proteomics is a powerful tool for identifying proteins associated with different disease states.

Traditional biomarker analysis concentrates on identifying one marker of a particular disease, usually based on an analysis of a known mechanistic pathway. Proteomics offers the ability to identify multiple markers simultaneously, and also to discover novel proteins not previously associated with particular disease states. For diagnostic and prognostic purposes, identification of biomarkers in readily obtainable samples, such as blood, sputum, saliva or urine, is required.

Limited studies in asthma and COPD have used proteomic methods to investigate serum protein expression in these diseases. Substantial differences in protein profiles between asthmatic and non-asthmatic pregnant women have been identified using SELDI-TOF mass spectrometry, however due to the nature of the technology used, the proteins of interest could not be identified (Murphy *et al.*, 2006). Recently, a selected analysis of multiple known markers in COPD using protein array methodology has further shown the potential for proteomics, whereby a panel of biomarkers were found to associate with COPD patients versus healthy controls (Pinto-Plata *et al.*, 2007). A drawback of this type of study is that the biomarkers identified are limited by the pool of analytes available and by our current knowledge of disease pathology. Furthermore, while comparison of COPD to healthy controls highlighted some potentially useful biomarkers, in the clinic there is a far greater need for indicators that distinguish between COPD and other respiratory diseases, such as asthma, as diagnosis will determine the different treatment course required for each specific disease. Thus identification of biomarkers in asthmatics compared to COPD patients is required.

Leru et al. (1998), Rom J Intern Med. 36(1-2):105-11 describe the study of fluticasone propionate efficacy in the treatment of patients with bronchial asthma not controlled by other inhaled corticosteroids.

Vysotnyuk et al. (2006), Likars'ka sprava no. 1-2: 65-81 investigate the use of fluimucyl-antibiotic therapy for patients with acute attack of infectious chronic obstructive lung disease.

Schoonbrood et al. (1994) Am J Respir Crit Care Med. 150 (6) Pt 1: 1519-1527 analyze plasma-protein leakage and local secretion in sputum from patients with asthma and chronic obstructive pulmonary disease.

Schoonbrood et al. (1995) Clin Chim Acta 240 (2): 163-78 investigate plasma protein leakage and local secretion of proteins assessed in sputum in asthma and COPD.

WO 2008/109730 A1 discloses a method of treating an inflammatory or fibrotic disease such as pulmonary disease.

Engström et al. (2009) THORAX 64: 211-215 investigate plasma markers of inflammation and incidence of hospitalisations for COPD based on a population-based cohort study.

WO 2006/105252 A2 provides genes and proteins that are regulated differentially in individuals with COPD, as well as methods for their use.

Sandford et al. (1997) Eur Respir J 10: 1380-1391 describe genetic risk factors for COPD.

Baker et al. (2000) Respiration 67: 533-538 investigate whether plasma or local production is the major source of ceruloplasmin in the lungs of patients with acute respiratory distress syndrome.

van Rensen et al. (2002) Am J Respir Crit Care Med 165: 1275-1279 assess microvascular leakage via sputum induction.

Zak-Nejmark et al. (1986) Archivum Immunologiae et Therapiae Experimentalis 34: 547-552 describe histamine binding proteins identified in healthy subjects and asthmatic patients.

Syromyatnikova et al. (1980) Therapeutic Archives, Moscow, RU, 52 (7): 36-37 describe clinical and biochemical findings in patients with nonspecific diseases of the respiratory organs.

## Summary of the Invention

As described herein the present inventors have quantified the contribution of a panel of biomarkers to disease diagnosis and have identified a series of biomarkers, the differential expression profiles of which enable the accurate diagnosis of, and between, asthma and COPD.

Accordingly, provided herein is a method for diagnosing an obstructive airways disease in a subject, the method comprising obtaining a biological sample from the subject and determining the level of expression of one or more proteins identified in Table 2 herein in the sample, wherein the expression profile of the one or more proteins in the sample is indicative of an obstructive airways disease.

In a first aspect there is provided a method for diagnosing an obstructive airways disease selected from asthma, chronic obstructive pulmonary disease (COPD) and mixed (asthma and COPD) disease in a subject, the method comprising determining the level of expression of ceruloplasmin and hemopexin, and optionally one or more proteins selected from haptoglobin and $\alpha$-2-macroglobulin, in an *in vitro* biological sample from the subject, wherein an elevation in the expression level of ceruloplasmin and hemopexin, and optionally one or more proteins selected from haptoglobin and $\alpha$-2-macroglobulin, in the *in vitro* biological sample from the subject compared to the expression level of ceruloplasmin and hemopexin, and optionally one or more proteins selected from haptoglobulin and $\alpha$-2-macroglobulin from one or more control samples, is indicative of said obstructive airways disease. The expression of one or more isoforms of the protein(s) may be determined.

Typically the obstructive airways disease is asthma or chronic obstructive pulmonary disease (COPD).

The sample may be derived from any suitable body fluid or tissue. For example the sample may comprise blood (such as whole blood, blood plasma or blood serum), sputum, saliva or urine. In a particular embodiment the sample comprises peripheral whole blood, blood plasma, serum or sputum.

Typically the expression profile of a protein in the sample from the subject is compared to the expression profile of the same protein from one or more control samples, or the combined expression profile of two or more proteins in the sample from the subject is compared to the combined expression profile of the same two or more proteins from one or more control samples, wherein abnormal expression profiles of the protein(s) in the sample from the subject compared to the one or more control samples is indicative of obstructive airways disease. Typically a control sample is derived from one or more individuals known not to suffer from an obstructive airways disease, or alternatively known to have an obstructive airways disease selected from asthma and COPD.

The method may be used for the diagnosis of airways disease in the subject. Alternatively, or in addition, the method may be used to determine if the subject, having an airways diseases, suffers from asthma or from COPD. Alternatively, or in addition, the method may be used to determine the asthma subtype of an asthma sufferer.

The expression of the one or more proteins may be determined by measuring expression at the protein or the nucleic acid level. Protein expression may be analysed and/or measured by any suitable means, including for example enzyme linked immunosorbent assay (ELISA), immunoblotting, two-dimensional gel electrophoresis, multiplex protein expression assays, flow cytometry and protein expression profiling arrays and microarrays. The two-dimensional gel electrophoresis may be two-dimensional difference gel electrophoresis (2D-DIGE).

The combined expression profile for any two or more proteins typically comprises determining the individual expression levels of the proteins. The combined expression profile may be subject to, or result from, statistical analysis, for example multiple regression analysis, meta-analysis and/or receiver operator curve (ROC) analysis.

In an embodiment an increase in the expression of ceruloplasmin and hemopexin in a sample from the subject relative to the expression of the corresponding protein in one or more control samples from individual(s) known not to suffer from an obstructive airways disease is indicative that the subject suffers from asthma.

In an embodiment an increase in the expression of ceruloplasmin and hemopexin, and optionally one or more proteins selected from haptoglobin and $\alpha$-2-macroglobulin in a sample from the subject relative to the expression of the corresponding protein in one or more control samples from individual(s) known not to suffer from an obstructive airways disease is indicative that the subject suffers from COPD.

In an embodiment, the expression of $\alpha$-2-macroglobulin is increased in a sufferer of COPD relative to one or more control samples from individual(s) with asthma. The combined expression profile of $\alpha$-2-macroglobulin in combination with ceruloplasmin, haptoglobin or hemopexin is also increased in sufferers of COPD as compared to sufferers of asthma.

In an embodiment the combined expression profile of one or more of the following groups of proteins is increased in a sufferer of asthma relative to one or more control samples from individual(s) known not to suffer from an obstructive airways disease:

> (i) ceruloplasmin and hemopexin;
> (ii) ceruloplasmin, haptoglobin and hemopexin; and
> (iii) ceruloplasmin, haptoglobin, hemopexin and $\alpha$-2-macroglobulin.

In an embodiment the combined expression profile of ceruloplasmin and hemopexin is increased in a sufferer of both asthma and COPD relative to one or more control samples from individual(s) known not to suffer from an obstructive airways disease.

The method may further comprise in step (b) determining the level of expression of one or more of the additional proteins identified in Table 2 herein.

In a second aspect there is provided a method for determining a treatment regime for a subject suffering from an obstructive airways disease selected from asthma, COPD and mixed (asthma and COPD) disease, the method comprising:

(a) determining the level of ceruloplasmin and hemopexin, and optionally one or more proteins selected from haptoglobin and $\alpha$-2-macroglobulin, in an *in vitro* biological sample from the subject, optionally further comprising comparing the expression level of the proteins in the *in vitro* sample from the subject to the expression level of the corresponding proteins from control samples;
(b) diagnosing the obstructive airways disease on the basis of the determination in (a); and
(c) selecting an appropriate treatment regime for the subject on the basis of the diagnosis.

In an embodiment of the second aspect the method comprises diagnosing asthma or COPD in a subject known or thought to suffer from an obstructive airways disease and selecting an appropriate treatment regime for the subject on the basis of the diagnosis.

In a third aspect there is provided a method for determining disease control in a subject suffering from an obstructive airways disease selected from asthma, COPD and mixed (asthma and COPD) disease, the method comprising:

(a) determining the level of ceruloplasmin and hemopexin, and optionally one or more proteins selected from haptoglobin and $\alpha$-2-macroglobulin in an *in vitro* biological sample from the subject; and
(b) repeating at least step (a) at least once over a period of time and determining whether the levels of the proteins change over the period of time and optionally,
(c) comparing the level of the proteins in the sample from the subject at one or more points in time to the expression profile of the corresponding proteins from one or more control samples. wherein changes in the expression profiles obtained in (b) and optionally (c) are indicative of disease control in the subject.

Also disclosed herein is a method for determining the efficacy of treatment regime for a subject with an obstructive airways disease, the method comprising: (a) treating the subject with a treatment regime for a period sufficient to evaluate the efficacy of the regime; (b) obtaining a biological sample from the subject; (c) determining the level of expression of one or more proteins selected from ceruloplasmin, haptoglobin, hemopexin, $\alpha$-2-macroglobulin, prothrombin, immunoglobulin A and complement factor H in the sample, and optionally comparing the expression profile of the one or more proteins in the sample from the subject to the expression profile of the corresponding proteins from one or more control samples; and repeating steps (b) and (c) at least once over the period of treatment, wherein a change in the level of expression of the one or more proteins over the period of treatment is indicative of efficacy of the treatment regime.

Also disclosed herein is a method for diagnosing an obstructive airways disease in a subject, the method comprising:

(a) obtaining a biological sample from the subject; and
(b) determining the level of expression of one or more proteins identified herein in Table 2 in the sample,

wherein the expression profile of the one or more proteins in the sample is indicative of an obstructive airways disease.

Also disclosed are methods for determining the asthma subtype in an asthma sufferer, comprising obtaining a biological sample from the asthma sufferer and determining the level of expression of one or more proteins listed in Table 2 herein in the sample, wherein the expression profile of the one or more proteins in the sample is indicative of the asthma subtype. In one embodiment, discrimination between neutrophilic and eosinophilic asthma can be achieved based on an altered expression profiles of the protein combinations listed in Table 15. In another embodiment discrimination between eosinophilic and paucigranulocytic ashma can be achieved based on the expression profiles of the proteins listed in Table 15. In another embodiment discrimination between paucigranulocytic and non-paucigranulocytic asthma can be achieved based on the expression profiles of the proteins listed in Table 15.

## Detailed Description of the Invention

There is now general agreement of the statistical argument that a panel of independent disease-related proteins considered in an aggregate should be less prone to the influence of genetic and environmental 'noise' than is the level of a single marker protein (Anderson, 2000), and that proteomics has the power to identify such panels of proteins in a high-throughput manner. There remains however a need to apply methods to quantify the added benefit of biomarker panels for disease assessment. As described herein, the inventors have applied meta-analysis of individual proteins to permit a quantitative assessment of the contribution made by a panel of markers to obstructive airways diseases. Consequently, described herein are the results of the first proteomic study of asthma and COPD in well characterised patients, assessed using powerful 2D-DIGE (two-dimensional difference gel electrophoresis) technology and the novel application of this method to quantify the contribution of a panel of biomarkers to disease diagnosis.

The inventors have identified circulating proteins that when assayed, typically in combination of two or more, are highly discriminatory for obstructive airways disease, in particular asthma, COPD and mixed obstructive airways disease. As used herein the term "mixed obstructive airways disease" refers to obstructive airways disease that is characterised by features of both asthma and COPD and/or in which sufferers display symptoms characteristic of, or otherwise associated with, both asthma and COPD.

The methodology involved screening the human plasma proteome from well characterised clinical groups, and then applying meta-analysis to provide a quantitative estimate of the discriminatory power of the protein diagnostic suite. The discriminatory proteins identified herein are selected from the proteins listed in Table 2. In particular embodiments the discriminatory proteins are selected from ceruloplasmin, haptoglobin, hemopexin, $\alpha$-2-macroglobulin, immunoglobulin A (IgA), complement factor H and prothrombin. More particularly, the discriminatory proteins are selected from ceruloplasmin, haptoglobin, hemopexin and $\alpha$-2-macroglobulin.

The work described herein has also identified that multiple isoforms of the discriminatory proteins are present in plasma, indicating a role for post-translational protein modification that may have functional implications in the mechanisms underlying asthma and COPD. Accordingly, embodiments disclosed herein contemplate the determination of expression of isoforms of the discriminatory proteins in the diagnosis of obstructive airways diseases.

Also in accordance with embodiments of the present disclosure is the determination of expression of precursors, derivatives, variants, analogues and functional fragments of the discriminatory proteins. A variant of a discriminatory protein includes molecules which exhibit at least some of the functional activity of the form of protein of which it is a variant. Exemplary amino acid sequences of the human proteins listed in Table 2 are provided in Table 2. In addition, sequences for proteins listed in Table 2, including variant forms, isoforms and precursors thereof can be readily ascertained by those skilled in the art, for example by reference to suitable databases such as the NCBI Entrez Protein database (www.ncbi.nlm.nih.gov). By way of example, representative sequences of human ceruloplasmin can be found under Accession No. AAA51975 and BAA08084, of human haptoglobin can be found under Accession No. AAA88080, of human hemopexin can be found under Accession No. AAA58678, of human $\alpha$-2-macroglobulin can be found under Accession No. AAA51552 and AAH40071, of human prothrombin can be found under Accession No. AAC63054, of human immunoglobulin A can be found under Accession No. AAB30803 and AAF78713, and of human complement factor H can be found under Accession No. CAA30403 and CAI19672.

The best currently available diagnostic marker for asthma is $\Delta$FEV1 post bronchodilator, with a sensitivity and specificity of 60% and 70% respectively (Johns and Crockett, 2005). It is exemplified herein that in a pre-clinical analysis the combination of the protein biomarkers IgA, prothrombin and complement factor H significantly improves the performance as a diagnostic marker (improving specificity and/or selectivity) than each individual protein alone. Indeed, the combined expression of IgA, prothrombin, and complement factor H achieves a sensitivity of 100% and specificity of 100% for distinguishing between COPD, asthma and healthy controls. Moreover in a further clinical validation of obstructive airways disease markers, the proteins ceruloplasmin, haptoglobin, hemopexin and $\alpha$-2-macroglobulin, both individually and in various combinations (as described and exemplified hereinafter) are demonstrated to provide sensitive and specific diagnoses between asthma and healthy controls, between COPD and healthy controls, between mixed disease (asthma and COPD) and healthy controls, and between COPD and asthma. Thus, ceruloplasmin, haptoglobin, hemopexin and $\alpha$-2-macroglobulin, alone or in pairwise or greater combinations, represent powerful and sensitive diagnostic indicators.

As the biomarkers disclosed herein are detectable in readily obtainable biological samples such as blood and sputum, and analytes are currently available for testing the abundance of these proteins, the panels of biomarkers described have the potential to become an extremely useful addition to current approaches to clinical diagnosis and management of respiratory disease.

Thus, disclosed herein for the first time are simple diagnostic indicators of obstructive airways diseases, facilitating not only an initial diagnosis of disease in a patient, but also an accurate diagnostic distinction between asthma and COPD, based on the determination of expression levels of discriminatory proteins as disclosed herein. Asthma and COPD differ in initial treatment, in response to treatment, and in prognosis. Misdiagnosis can, for example, deny effective treatment to people with asthma, or lead to the over treatment of patients with a COPD. The ability to reliably distinguish between asthma and COPD is therefore critical in providing information about likely prognosis and response to treatment.

It should be understood that reference to determining the level of expression of a protein is intended as a reference to the use of any suitable technique which will provide information in relation to the level of expression of the desired protein in the relevant tissue of the subject. Accordingly, these techniques include both *in vivo* techniques, as well as *in vitro* techniques which are applied to a biological sample extracted from the subject. Such *in vitro* techniques are likely to be preferred due to their significantly more simplistic and routine nature.

Thus, in one aspect the invention provides a method for diagnosing an obstructive airways disease selected from asthma, chronic obstructive pulmonary disease (COPD) and mixed (asthma and COPD) disease in a subject, the method comprising determining the level of expression of ceruloplasmin and hemopexin, and optionally one or more proteins selected from haptoglobin and α-2-macroglobulin, in an *in vitro* biological sample from the subject, wherein an elevation in the expression level of ceruloplasmin and hemopexin, and optionally one or more proteins selected from haptoglobin and α-2-macroglobulin, in the *in vitro* biological sample from the subject compared tso the expression level of ceruloplasmin and hemopexin, and optionally one or more proteins selected from haptoglobin and α-2-macroglobulin from one or more control samples, is indicative of said obstructive airways disease.

In an embodiment an increase in the expression of ceruloplasmin and hemopexin in a sample from the subject relative to the expression of the corresponding protein in one or more control samples from individual(s) known not to suffer from an obstructive airways disease is indicative that the subject suffers from asthma.

In an embodiment an increase in the expression of ceruloplasmin and hemopexin, and optionally one or more proteins selected from haptoglobin and α-2-macroglobulin in a sample from the subject relative to the expression of the corresponding protein in one or more control samples from individual(s) known not to suffer from an obstructive airways disease is indicative that the subject suffers from asthma.

In an embodiment, the expression of α-2-macroglobulin is increased in a sufferer of COPD relative to one or more control samples from individual(s) with asthma.

In an embodiment the combined expression profile of one or more of the following groups of proteins is increased in a sufferer of asthma relative to one or more control samples from individual(s) known not to suffer from an obstructive airways disease:

(i) ceruloplasmin and hemopexin;

(ii) ceruloplasmin, haptoglobin and hemopexin; and

(iii) ceruloplasmin, haptoglobin, hemopexin and α-2-macroglobulin.

In an embodiment the combined expression profile of any two or more of ceruloplasmin and hemopexin is increased in a sufferer of asthma and COPD relative to one or more control samples from individual(s) known not to suffer from an obstructive airways disease.

Methods in accordance with embodiments of the invention may further comprise determining the level of expression of one or more of the additional proteins identified in Table 2.

Also provided are methods for determining a treatment regime for a subject suffering from an obstructive airways disease, the methods comprising, in an embodiment, diagnosing in accordance with the invention asthma or COPD in a subject known or thought to suffer from an obstructive airways disease and selecting an appropriate treatment regime for the subject on the basis of the diagnosis.

To improve asthma, clinical management needs objective measurement for inflammatory phenotype. However, the most direct measures of airway inflammation are too invasive and have limited clinical use. By using the proteomic methodologies described herein the present inventors have also identified that combinations of the proteins listed in Table 2 act as markers of asthma inflammatory phenotype or subtype, and thus can also be employed to predict and monitor patient response to therapeutic intervention. Accordingly, disclosed herein are methods for determining the asthma subtype experienced by an asthma sufferer, the method comprising determining the level of expression of any one or more of the proteins listed in Table 2 in a biological sample from a subject suffering from asthma. In particular embodiments the expression profiles of proteins and combinations of proteins as listed in Table 15 may be used to distinguish inflammatory subtypes of asthma. Accordingly, also disclosed herein and provided by embodiments of the invention are methods for determining appropriate treatment regimes and determining the efficacy of treatment regimes for specific asthma inflammatory phenotypes or subtypes, based on the analysis of expression profiles of proteins and combinations of proteins as listed in Table 15.

Thus, in particular embodiments, combinations of expression profiles for two or more of complement 4a, complement 3, complement factor B, antithrombin, ceruloplasmin, haptoglobin and hemopexin may be used for the discrimination between paucigranulocytic asthma and non- paucigranulocytic asthma, between eosinophilic asthma and neutrophilic asthma, between eosinophilic asthma and paucigranulocytic asthma, and between neutrophilic asthma and paucigranulocytic asthma.

Whilst particularly suitable for diagnosis in humans, it will be appreciated by those skilled in the art that methods embodied by the present invention are not limited thereto and may be extended to any mammal, for example a mammal useful as a model of an obstructive airways disease in humans.

Throughout this specification and the claims which follow, unless the context requires otherwise, the word "comprise", and variations such as "comprises" or "comprising", will be understood to imply the inclusion of a stated integer or step or group of integers or steps but not the exclusion of any other integer or step or group of integers or steps.

The articles "a" and "an" are used herein to refer to one or to more than one (*i.e.*, to at least one) of the grammatical object of the article. By way of example, "an element" means one element or more than one element.

As used herein the term "protein" means one or more peptide or polypeptide molecules having a particular function. As such the term "protein" encompasses not only the peptide or polypeptide product of a gene, but also functionally equivalent fragments, derivatives and variants thereof. Different isoforms of a protein are also encompassed by this general term. Similarly, the term protein encompasses different forms of a given protein, for example including and absent any post-translational modifications.

As used herein the term "expression profile" refers to the expression level of a protein in a given sample. The term "combined expression profile" refers to the expression profiles of two or more proteins in a sample. The expression profile for any given protein typically comprises the expression level of the protein, measured for example by any suitable method for determining, and typically quantifying, protein expression known to those skilled in the art. Alternatively expression may be measured at the level of mRNA or gene expression, also using techniques and methods known to those skilled in the art. Thus the expression or the expression profile for a given protein or proteins may be determined as the expression of a translational product or a transcriptional product. The person of skill in the art will determine the most appropriate means of analysis in any given situation. In particular embodiments, expression may be determined at the level of protein molecules due to the relative simplicity of the techniques which are likely to be utilised. Nevertheless in certain situations, and in the context of particular biological samples, it may be desirable or otherwise useful to directly analyse gene transcription. The nature of the technique which is selected for use may, in part, be determined by the type of biological sample which is required for analysis. Such determinations are well within the scope of the person of skill in the art.

In the context of the present disclosure, reference to an increase in an expression profile or combined expression profile in a given sample means an increase in the level of expression of the protein(s) in question in the sample, typically when compared to the expression levels in one or more control samples. The expression profile employed in diagnostic methods disclosed herein may be subject to, or may result from statistical analysis of expression levels, for example, in comparing expression profiles between samples including control samples. Statistical techniques that may be employed for such analyses are known to those skilled in the art and include, but are not limited to, meta-analysis, multiple regression analysis, and receiver operator curve (ROC) analysis. ROC analysis is used to determine a score that is diagnostic with the greatest sensitivity and specificity. The 'squarer' the look of the curve, the simpler the determination of a diagnostic level or score. The closer the area under the curve is to 1 also indicates a result with high sensitivity and specificity.

As used herein the term "treatment" refers to any and all treatments which remedy a condition or one or more symptoms of a condition or disease, prevent the establishment of a condition or disease, or otherwise prevent, hinder, retard, or reverse the progression of a condition or disease or other undesirable symptoms in any way whatsoever. Thus the term "treatment" is to be considered in its broadest context. For example, treatment does not necessarily imply that a patient is treated until total recovery.

Based on the surprising finding described herein that proteins selected from ceruloplasmin, haptoglobin, hemopexin, α-2-macroglobulin, immunoglobulin A, prothrombin, and complement factor H can be used to improve selectivity and specificity of diagnosis of obstructive airways diseases such as asthma and COPD, embodiments of the present invention find application in diagnosing asthma and COPD in individuals, for example in those who may present with one or more symptoms of asthma or COPD but in whom the disease has not been confirmed. Further embodiments of the invention find application in the discrimination between inflammatory phenotypes (or subtypes) of asthma (including neutrophilic, eosinophilic, paucigranulocytic and non-paucigranulocytic) in subjects with asthma.

Embodiments of the invention may therefore be used alone or more typically in conjunction with, or as an adjunct to, one or more other diagnostic methods and tests to determine the likelihood that an individual may suffer from asthma or COPD or to diagnose the disease, or to diagnose an asthma inflammatory phenotype or subtype. Such other diagnostic methods and tests will be well known to those skilled in the art.

Embodiments of the invention contemplate the determination and measurement of expression of one or more forms of the discriminatory proteins disclosed herein. Thus, the diagnosis of obstructive airways diseases and subtypes thereof in accordance with embodiments of the present invention may be based upon the detection and/or quantification of whole protein, protein fragments and/or individual isoforms of one or more of the discriminatory proteins disclosed herein.

The protein expression profiles determined in samples from subjects in accordance with methods of the invention can be compared to control values as a suitable reference to assist in diagnosis. For example, for control purposes expression profiles of protein biomarkers as described herein may be determined in one or more, typically a population, of individuals not suffering from an obstructive airways disease. Alternatively or in addition, the protein expression profiles may be determined in a range of individuals with different obstructive airways diseases, such as asthma or COPD. Similarly, for the diagnosis of asthma subtypes or inflammatory phenotypes, control expression profiles may be derived from individuals with different asthma subtypes or inflammatory phenotypes, depending on those subtypes and inflammatory phenotypes to be discriminated or diagnosed. In subjects to which methods of the invention are applied, a comparison of the protein expression profiles with those obtained from the appropriate reference or control may determine diagnosis.

Reliable diagnoses of asthma and COPD, and of asthma subtypes or inflammatory phenotypes, such as are possible utilising methods of the invention also facilitate decision making with respect to the most appropriate intervention or treatment regime for individual subjects. The treatment regime may be tailored not only to the specific disease or disease subtype suffered by the subject but also to the subject themselves based on one or more other factors such as the severity of the symptoms, the subjects lifestyle, age, weight, general health etc. For example, this may comprise introducing a new treatment regime or modifying an existing regime employed by the subject. The modification of a regime may be modification with respect to any one or more of a variety of factors, such as the nature of any anti-asthma or anti-COPD medication, the dosage thereof, the timing of administration and/or any supplementary management strategies. Such decision making with respect to treatment regimes will vary from case to case and the determination of the most appropriate strategy is well within the expertise and experience of those skilled in the art.

Those skilled in the art will readily appreciate that in accordance with embodiments of the invention protein expression may be determined by any suitable technique or assay known in the art. For example, expression of a protein may be determined, for example, by immunoassay using an antibody(ies) that bind with the protein. Suitable techniques and methodologies for determining protein expression include, for example, enzyme-linked immunosorbent assay (ELISA), immunoblotting, 2D-gel electrophoresis (including 2D-DIGE), multiplex protein expression assays, flow cytometry and protein expression profiling arrays and microarrays. Alternatively or in addition protein expression may be determined at the level of the nucleic acids encoding the proteins, such as by RT-PCR and qRT-PCR. Where protein expression is detected and/or measured using antibody-based techniques, the antibody may be a polyclonal antibody, a monoclonal antibody, or an antigen-binding fragment thereof. The skilled addressee will appreciate that the invention is not limited by reference to the means by which protein expression is determined and/or quantified.

A treatment regime for the treatment of asthma or COPD, or an asthma subtype or inflammatory phenotype, in a subject in accordance with a method of the invention may involve administration of any of the medications commonly utilised in the treatment of the disease. The treatment regime may comprise the administration of a number of drugs simultaneously, sequentially, or in combination with each other or with non-drug treatments. The type of drug(s) administered, dosage, and the frequency of administration can be determined by medical physicians in accordance with accepted medical principles, and will depend on factors such as the severity of the disease, the age and weight of the subject, the medical history of the subject, other medication being taken by the subject, existing ailments and any other health related factors normally considered when determining treatments for obstructive airways disease.

The present disclosure also provides kits suitable for use in accordance with the methods of the invention. Such kits include for example diagnostic kits for assaying biological samples, comprising an agent(s) for detecting expression levels discriminatory proteins disclosed herein or encoding nucleic acid molecules, and reagents useful for facilitating the determination of expression by the agent(s). The agent(s) may be any suitable detecting molecule. Kits may also include other components required to conduct the methods of the present invention, such as buffers and/or diluents. The kits typically include containers for housing the various components and instructions for using the kit components in the methods of the present invention.

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates. The present invention will now be described with reference to the following specific examples, which should not be construed as in any way limiting the scope of the invention.

**Examples**

***Chemicals and Reagents***

ProteomeLab IgY-12 LC2 proteome partitioning kit was purchased from Beckman Coulter, Inc. (Fullerton, CA, USA). CyDye DIGE Fluors Cy2, Cy3 and Cy5 (minimal dye), Immobiline Drystrips (pH 4-7, 24 cm) PlusOne™ drystrip cover fluid, Pharmalyte™ 3-10 for IEF, Bind-silane solution, 2D quant kit, ECL anti-mouse IgG HRP-linked whole antibody (from sheep), ECL anti-rabbit IgG HRP-linked whole antibody (from sheep), Hybond® C-extra nitrocellulose and ECL Plus western blotting detection system were purchased from GE Healthcare Bio-Sciences AB, Uppsala, Sweden. SDS, 40% acrylamide solution, 30% acrylamide/Bis solution (37.5:1, 2.6%C), SyproRuby™ protein stain and SDS PAGE standards (broad range, unstained) were all electrophoresis purity and purchased from BioRad, NSW, Australia. Agarose (Type I-A: Low EEO), tributyl phosphine solution (TBP), N,N,N',N'-tetramethylethylenediamine (TEMED, >99%), $CF_3COOH$ (TFA, Fluka Biochimika, >99.5%) $NH_4HCO_3$ (Reagent Plus®, ≥ 99%), $CCl_3COOH$ (TCA, 99%), $L$-lysine monochloride (> 98%), thiourea (ACS reagent), CHAPS (> 98%), bovine serum albumin (BSA) Fraction V, protease inhibitor cocktail, and anti-goat/sheep-HRP were purchased from Sigma-Aldrich, NSW, Australia. $(NH_4)_2S_2O_8$ (>98 %), urea (>98 %), glycine (>98.5%) were sourced from Chem. Supply, Gillman, SA, Australia. Tris(hydroxymethyl)-aminomethane (ACS reagent), glycerol (BDH AnalR, > 99.5%), $CH_3CN$ (BDH HiPer Solv for HPLC, >99.9 %), NaOH (BDH

AnaIR, 99%) and $CH_3COOH$ (BDH, 100%) were purchased from Merck Australia, VIC, Australia. Methanol ($CH_3OH$, > 99.8%) was purchased from Ajax Finechem., NSW, Australia. Dithiothreitol (DTT, >99.5%) was purchased from Applichem, Darmstadt, Germany. Bromophenol blue (BPB) was purchased as the sodium salt from Research Organics, Cleveland, OH, USA. $(CH_3)_2NCOH$ (DMF, ≥99.5%) was purchased from USB Corporation, Cleveland, OH, USA and was stored in the dark under a $N_2$ atmosphere. □-cyano-4-hydoxycinnamic acid (□-CHCA, recrystallised) and peptide calibration mix 1 (1000-2500 Da) were purchased from Laser BioLabs, Sophia-Antipolis Cedex, France. Sequencing-grade modified porcine trypsin was purchased from Promega (Madison, WI, USA). Ziptip® μ-C18 pipette tips and 0.45 □m white nylon filters were purchased from Millipore. MA, USA. Human IgA ELISA quantitation kit and ELISA starter accessory package were purchased from Bethyl Laboratories, Inc., TX, USA. Anti-prothrombin (ab48627) was purchased from Abcam, Cambridge, UK and anti-factor H (L20/3): sc-47686 mouse monoclonal antibodies were purchased from Santa Cruz Biotechnology, Inc., Ca, USA. All reagents were used as received without further purification.

### Part 1 - Pre-clinical study

#### *Subject recruitment*

Adults with stable asthma (n=20) and COPD (n=5) were recruited from the John Hunter Hospital ambulatory care clinics. Healthy age and sex matched non-asthmatic controls (n=17) were recruited from existing research databases. Asthma was defined by clinical history and evidence of reversible airflow obstruction with airway hyperresponsiveness (AHR) to hypertonic saline (4.5%). COPD (GOLD stage II or greater) was defined as incompletely reversible airflow obstruction. Stable airway disease was defined as no increase in bronchodilator use, no use of oral corticosteroids, no limitation in activities, no doctor's visit and no hospitalisation due to asthma in the past 4 weeks. Subjects with recent respiratory tract infection (past 4 weeks) were excluded. The study was approved by the Hunter Area Health Service and University of Newcastle Human Research Ethics Committees and subjects gave written informed consent.

#### *Clinical Assessment and Characterisation*

Subjects were assessed following an overnight fast (minimum 12 hours). Peripheral blood was collected from a vein in the forearm. Sputum was induced during hypertonic saline challenge and a differential cell count was used to determine inflammatory phenotype. Table 1 describes the recruitment criteria that were established to clinically characterise the subject groups of interest, with the aim of establishing mutually exclusive clinical diagnostic groups of asthma, COPD, and controls without airway disease. None of the subjects were current smokers. The recruitment criteria ensured there was control for smoking and that there was control for age and sex effects by matching subjects within defined age ranges.

Table 1- Clinical groupings

| Group | 1 | 2 | 3 | 4 | 5 |
|---|---|---|---|---|---|
| Description | Healthy Controls (NS) | Healthy Controls (ExS) | Stable Asthma (NS) | Stable Asthma (ExS) | COPD (ExS) |
| Age (Years) | 39.6 ± 4.3 | 47.4 ± 5.2 | 47.5 ± 4.2 | 47.8 ± 3.8 | 65.4 ± 5.1 |
| Sex (Male/ Female) | 4/4 | 4/5 | 6/5 | 5/5 | 2/3 |
| Pack Years (Exsmokers) | N/A | 20.3 ± 5.8 | N/A | 20.7 ± 4.2 | 72.7 ± 16.5 |
| %predicted $FEV_1$[a] | 99.5 ± 3.2 | 96.1 ± 3.0 | 83.2 ± 4.5 | 74.9 ± 4.6 | 64.5 ± 7.8 |
| %predicted FVC[a] | 100.2 ± 4.1 | 102.2 ± 3.9 | 97.6 ± 5.0 | 90.1 ± 3.9 | 79.9 ± 4.9 |
| $FEV_1$/FVC %[a] | 82.4 ± 3.1 | 76.9 ± 2.3 | 70.2 ± 2.2 | 68.1 ± 2.5 | 55.0 ± 12.9 |
| $PD_{15}$(mL)[c] | N/A | N/A | 0.65 ± 0.18 | 0.98 ± 0.13 | 0.75 ± 0.09 |

(continued)

| Group | 1 | 2 | 3 | 4 | 5 |
|---|---|---|---|---|---|
| $D_{LCO}$ | N/A | N/A | 88.6 ± 5.4 | 83.4 ± 5.1 | 65.6 ± 5.1 |
| ICS ($\mu$g beclamethasone equivalents /day)[b] | 0 | 0 | 1000 (400, 1000) | 800 (500, 1000) | 0 |
| Number of patients | 8 | 9 | 11 | 10 | 5 |

[a]Values are Mean ± SEM; [b]Values are median (interquartile range); [c]$PD_{15}$ is provocation dose resulting in 15% drop in baseline $FEV_1$ expressed as geometric mean (log SD); $FEV_1$ is forced expiratory volume in 1 second; FVC is forced vital capacity; NS is never smoker; ExS is ex-smoker; COPD is chronic obstructive pulmonary disease; $D_{LCO}$ is carbon monoxide diffusing capacity; ICS is inhaled corticosteroids.

The COPD group were selected to have COPD with predominant airway disease with minimal emphysema (DLCO>70% predicted) and no asthma. They were ex-smokers with a significant smoking history, compatible symptoms and a doctors diagnosis of COPD, and incompletely reversible airflow obstruction (FEV1<80% predicted, FEVNC < 0.7). In addition, there was no evidence of asthma since they had no AHR and a negative bronchodilator response (BDR). An additional group of ex-smokers with COPD were also recruited to enlarge the sample size of the study group. Asthma was defined as a compatible history of episodic wheeze, cough and dyspnea that were responsive to asthma treatment, together with airway hyperresponsiveness to hypertonic (4.5%) saline. They had fully reversible airflow obstruction and no evidence of COPD as FEVNC ratio after bronchodilator was normal (>70%) and DLCO was >70% predicted. Healthy controls had no respiratory symptoms nor a diagnosis of respiratory disease, together with normal measures of airway function. Atopy was assessed using skin prick testing to common allergens. Airway responsiveness was assessed using hypertonic saline (4.5%) provocation challenge as described.

***Sample collection and immunodepletion***

Blood samples were collected from a peripheral vein in the forearm. Samples were centrifuged at 2000g for 10 minutes at room temperature. Plasma was removed and centrifuged at 2500g for 15 minutes at room temperature to deplete platelets. Protease inhibitor cocktail (Sigma-Aldrich, Missouri, USA) (1% v/v) was added to the platelet depleted plasma prior to storage at -80°C. Each plasma sample was subjected to immuno-depletion using an IgY-12 LC-2 Proteome Partitioning Kit (Beckman Coulter™, CA, USA) and an AKTA P920 FPLC system (GE Healthcare Bio-Sciences AB, Uppsala, Sweden). Using this methodology, the twelve most abundant plasma proteins (albumin, IgG, $\alpha$1-antitrypsin, IgA, IgM, transferrin, haptoglobin, $\alpha$1-acid glycoprotein, $\alpha$-2-macroglobulin, HDL (apolipoproteins A-I and A-II) and fibrinogen) are depleted from samples to yield a sub-sample for analysis that was enriched with respect to the other proteins present (Liu *et al,* 2006). Briefly, human plasma (50 $\mu$L) was diluted in dilution buffer (200 $\mu$L, 10 mM Tris-HCl pH 7.4, 150 mM NaCl), centrifuged at 8300 rpm for 1 min and loaded into an injection loop. The IgY column was pre-equilibrated (dilution buffer, 1 column volume (CV), 0.35 mL min$^{-1}$) prior to automated sample injection. The unbound protein fraction was collected (fraction 1, 5 mL) using a Frac 900 fraction collector. Dilution buffer was passed through the column (3.5 CV, 0.35 mL min$^{-1}$) prior to elution of the bound (high abundance) proteins with stripping buffer (50 mM glycine-HCl, pH 2.5, 8 CV, 0.35 mL min$^{-1}$). The eluate was subsequently neutralised with 10 x neutralisation buffer (1 M Tris-HCl, pH 8.0) and stored at -80 °C until required. The column was then immediately re-equilibrated with dilution buffer (32 CV, 0.35 mL min$^{-1}$) to neutralise the column pH. As 100$\mu$g of protein was required per sample hence multiple depletion runs (three to four) were performed and pooled in order to obtain the required quantity of protein. Eluted proteins were stored immediately at -80 °C until required. The unbound column fractions were thawed, replicates pooled and subsequently concentrated by TCA precipitation. Briefly, an aqueous stock solution of TCA (90% $^w$/v) was added to each of the pooled replicates to yield a final concentration of 9% and the samples were left overnight at 4 °C to facilitate protein precipitation. Each tube was then centrifuged at 15,000 x g (15 min, 4 °C) and the supernatant discarded. The protein pellet was washed sequentially in acidified ethanol (40 mM $CH_3COOH$ in $CH_3OH$) and ice-cold acetone (precipitate pelleted via centrifugation at 15,000 xg (15 min, 4 °C)), dried in a rotary vacuum concentrator. Proteins were allowed to precipitate overnight at 4 °C, centrifuged for 15 min at 15000 g (4 °C) and the resultant pellet resuspended in DIGE lysis buffer (30 mM Tris pH 8.5, 2M thiourea, 7M urea, 4%(w/v) CHAPS, pH 8.5). Samples were vortexed briefly and pulse sonicated (2 x 2 second pulse at an amplitude of $\leq$ 2 microns whilst on ice) prior to analysis. Sample pH was adjusted to 8.5 with NaOH solution (50 mM) and the quantity of protein recovered estimated using a 2-D Quant Kit (GE Biosciences) according to the manufacturer's recommendations.

### 2D-DIGE

An experimental design was formulated in order to test the reproducibility of the sample preparation and 2D-DIGE technique. Plasma was collected from one healthy control (#9173) and aliquoted into 3 separate samples (labelled A, B, C). Each sample was immunodepleted, TCA precipitated, and quantitated as described above. Reproducibility of the FPLC has been demonstrated (data not shown). Briefly, each sample was then separated into 3 aliquots labelled with Cy2, Cy3 or Cy5. Equal protein from the Cy2 samples were pooled for the internal control to be run on every gel. The remaining samples were separated by 2D-DIGE in two independent gel runs. The variability between dye labelling, sample preparation, and gel runs was determined across all spots using the DeCyder generated volume ratios (see below).

For the biomarker study, an experimental design was formulated with the 43 samples assigned to a particular gel and CyDye™ label. Within each clinical group, some samples were labelled with Cy3 and others the Cy5. An internal pooled control was produced from equal quantities of ten of the samples (two per clinical group). Proteins were CyDye labelled and separated on 24cm pH 4-7 IPG strips in the first dimension and 4-18% gradient acrylamide gels in the second dimension as previously described (Verrills *et al*, 2006). Protein separations were visualised on a Typhoon 9410 Variable Mode Imager with Ettan DALT alignment guides using excitation/emission wavelengths specific for Cy2 (488/520 nm), Cy3 (532/580 nm) and Cy5 (633/670 nm) The photomultiplier tube voltage (PMT) was adjusted to preclude spot intensity saturation. The resultant image files were cropped using the program ImageQuant™ Tools 2.1 and saved using the DIGE file naming format.

### 2D-DIGE Image analysis

Image analysis was performed using the Batch Processor and Biological Variation Analysis (BVA) modules of the DeCyder 2D software version 6.5 (GE Healthcare, Australia). This analysis first normalizes each sample to its respective in-gel Cy2 internal standard, and then matches all controls and samples between different gels. A total of 1918 spots were detected in the master gel (automatically assigned by DeCyder) and matched across the gel images. Comparing each disease group in the BVA module generated average expression ratios and Student's t tests of individual protein spots. One way analysis of variance (1-ANOVA) was also used to identify protein spots that showed a significant change across the 5 disease groups. Proteins with an expression ratio of +/-1.2 (assigned following reproducibility analysis - see results) and $p < 0.05$ by 1-ANOVA or individual group comparison Student's *t*-test, were assigned as a protein of interest (POI). Each of these proteins spots were manually inspected for densitometric Gaussian distribution and match quality. POI's were exported into the extended data analysis (EDA) module of the DeCyder software package, and protein markers determined by K-nearest neighbour discriminant analysis. This analysis determines the least number of protein spots whose combined expression pattern discriminates between each disease group.

### Meta-Analysis

The protein spots identified by K-nearest neighbour discriminant analysis were combined in a meta-analysis for each of the comparisons asthma vs healthy, COPD vs healthy and COPD vs asthma. The three most significant proteins from EDA analysis (1001, 822, 349) were combined as discrete subgroups and pooled to create a composite score using a standardised mean difference (SMD). Comparisons were undertaken in a manner which resulted in all differences being a positive value so that the magnitude of differences between disease groups to be assessed. The SMD is defined as the quotient of the mean difference and standard deviation (www.cochrane-net.org/openlearning). Multiple logistic regression analysis was also used to derive estimates of sensitivity and specificity for multiple markers used in combination. Receiver operator curve (ROC) analyses were conducted for each protein spot and for protein combinations to evaluate the change in protein average ratio that is diagnostic for each disease comparison.

### Protein Identification

For protein identification, five preparative 2D-gels were prepared, one from each disease group. Immuno-depleted plasma (600 μg) was separated by 2D-PAGE as described above. Gels stained with SyproRuby™ protein stain or Colloidal Coomassie G250 and protein spots of interest excised subjected to tryptic digestion as previously described (Verrills *et al*, 2006). Tryptic digests were analysed by MALDI-TOF mass spectrometry. Peptide mass fingerprinting data was acquired using an Ettan MALDI-ToF/Pro mass spectrometer and analysed using the NCBI FASTA human database provided with the system. The instrument was operated in positive reflectron mode acquiring duplicate spectra (700 - 4000 *m/z*), each comprising signal from 400 laser shots (fixed laser power, 337 nm nitrogen laser). Positive ions were extracted into the mass analyser at 20 kV using pulsed extraction. The instrument was calibrated using Pepmix 1 (Laser Biolabs, Ang II (1046.542 Da) and hACTH 18-39 (2465.199 Da)) according to the manufacturers directions.

Each sample spectrum was further internally calibrated using Trypsin I (842.508 Da) and trypsin III (2211.108 Da) autolytic peaks prior to database searching. Tryptic digestion was specified with only 1 missed cleavage allowed. The partial amino acid modifications oxidation (M) and propionamide (C) were considered and a fragment mass tolerance of 0.2 *m/z* allowed. PMF data was searched against *Homo sapiens* entries in the NCBI FASTA database (20060322) with the protein database update tool v1.30 using the PROFOUND peptide mass fingerprinting search engine licensed from ProteoMetrics (NY, USA). Bayesian statistics were used to rank the protein sequences in the database according to their probability of generating the experimental data (expt < 0.010 significant with p<0.05). Proteins were assigned as positive identifications if they showed an expectation value <0.05. Samples that were unable to be identified by MALDI-TOF were analysed by MALDI-TOF/TOF at the Australian Proteome Analysis Facility on an Applied Biosystems 4700 Proteomics Analyser with TOF/TOF optics in MS mode as previously described (Verrills *et al*, 2006). Data was submitted to the database search program Mascot (Matrix Science Ltd, London, UK) and searched against *Homo Sapien* entries in the Swisprot database. Significant Mascot scores in the database search indicated a likely match, and were confirmed or qualified by operator inspection.

## IgA ELISA

Non-depleted serum from forty-two of the patients in the DIGE study was tested for IgA concentration using a quantitative two-site ELISA kit (Bethyl Laboratories, Inc, TX, USA) as per manufacturers instructions. Briefly, Nunc Maxisorp C well strips were coated with the capture antibody goat anti-human IgA-affinity purified (100 $\mu$L, 1/10 dilution in coating buffer (0.05 M $CO_3^{2-}$/$HCO_3^{-}$, pH 9.6), 60 min). Post washing with 50 mM TBST, pH 8.0 (triplicate), residual binding sites on the ELISA plate wells were blocked with a post coat solution (1% BSA in 50 mM TBST, pH 8.0, 200 $\mu$L, 60 min) and then washed again in triplicate prior to antigen addition. Either human serum (100 $\mu$L, 1/27000 dilution in TBST) or known standards (100 $\mu$L) were added to the wells, incubated for 60 min followed by thorough washing (x5). Goat anti-human IgA-HRP conjugate (100 $\mu$L, 1:150,000 dilution in conjugate diluent (1% BSA in 50 mM TBST)) was added and incubated for 60 min prior to thorough washing (x5) and 3,3',5,5'tetramethylbenzidine (TMB)-$H_2O_2$ substrate addition (100 $\mu$L), 5 minute colour development and reaction termination (addition of 100 $\mu$L 2M $H_2SO_4$). Absorbance at 450 nm was read using a FLUOstar OPTIMA plate reader (BMG Labtechnologies, Offenburg, Germany). Both samples and standards were analysed in triplicate wells for each determination and the quantification was performed in triplicate (three separate runs). Triplicate absorbance values were averaged for each sample and a plot of $\log_{10}$ [IgA] vs absorbance (450 nm) constructed using GraphPad Prism® 4.02 for Windows. The standard curve was fit with a four parameter logistic curve-fit and individual sample [IgA] calculated via interpolation. The triplicate [IgA] determinations for each sample were averaged and data analysed for differentiation between known subject groupings using GraphPad Prism® 4.02 for Windows. Paired t-test analysis was used to test for statistical significance of the observed experimental trends.

## Western Blotting

Blood serum from forty-two of the donors in the DIGE study was analysed via Western blotting. Proteins were separated on 10 % acrylamide gels using a CBS Scientific Co. triple-wide electrophoresis tank (100 V, constant voltage), and transferred to nitrocellulose membrane using either a BioRad Trans Blot cell or CBS triplewide transfer tank (500 mA for 2 hr) with cooling. Membranes were blocked with 2 % BSA in Tris-buffered-saline with Tween (TBST, 20 mM Tris-Cl, 140 mM NaCl, pH 7.5, 0.05 % Tween-20) for one hour at room temperature or overnight at 4 °C. Primary antibodies anti-prothrombin (1:200 dilution in TBST) and anti-factor H (1:100 dilution in TBST) were incubated with the blocked membranes with agitation for 60 min at room temperature, the membranes washed in TBST (triplicate, 20 min) and then incubated with secondary antibody (sheep anti-mouse IgG, HRP-linked whole antibody, 1:5000 in TBST for Factor H; rabbit anti-mouse IgG, HRP-linked whole antibody, 1:10000 in TBST for prothrombin, 60 min) with agitation. Reactive bands were visualised using ECL chemiluminescent reagents and a Fujifilm Luminescent Image Analyser LAS-300. Immunoreactive bands were quantified using the software Multi Gauge V3.0 and the chemiluminescent intensity normalised to a serum sample (not included in the current analysis) to facilitate accurate quantitation. The blots were run in triplicate, normalised intensity values for each sample averaged and data analysed for differentiation between known subject groupings using GraphPad Prism® 4.02 for Windows. Unpaired Students t-test was used to test for statistical significance between the clinical groups.

## Statistical analysis for quantitative assessment of multiple biomarkers

Logistic regression was used to calculate the predicted value of an individual having the disease based on their level of a particular marker (simple logistic regression) or combination of markers (multiple logistic regression). The regression equation utilised was:

$$\ln(p/(1-p)) = \beta_0 + \beta_i x_i$$

$$p = \exp(\beta_0 + \beta_i x_i)/(1 + \exp(\beta_0 + \beta_i x_i))$$

The predicted value is the probability that the person has the disease given the level of the markers. Receiver-Operating Characteristic (ROC) Curves were generated from the predicted values from the regression analyses. The ROC curves were then used to determine the sensitivity and specificity of both individual and combinations of markers at specified cut points of the predicted values. An individuals' protein measurements can be entered into the logistic regression equation to determine their predicted value, a value above or below the cut point would determine their likelihood of disease with the defined sensitivity and specificity calculated from the ROC curve. The area under the curve (AUC) was also calculated. The AUC value gives a measure of the ability of the test to correctly classify people.

The significance of the regression models were used to assist in determining the best combination of markers. Sensitivity and specificity were used to describe the discriminatory power of the protein combinations. Cut-points were selected in order to maximise the discriminatory power in any given diagnostic situation.

**Example 1 - Differential protein expression in asthmatic and COPD patients**

Plasma proteins were separated by 2D-DIGE for 43 subjects across the 5 clinical groups: 1, healthy controls never smoked; 2, healthy controls ex-smokers; 3, asthmatics never smoked; 4, asthmatic ex-smokers; 5, COPD ex-smokers. Over 1900 proteins were detected and the relative abundance of each protein spot was compared between the clinical groups using DeCyder 2D Image Analysis. Of these proteins, 55 displayed a significant change in expression between the clinical groups according to 1-ANOVA. These proteins were subjected to MALDI-TOF MS, and 38 proteins were positively identified. Each of these proteins displays a unique pattern of expression, with a significant change in abundance between at least 2 disease groups.

Of the 38 individual protein spots, a number of these are isoforms of the same protein. For example, 13 individual protein spots were all identified as immunoglobulin alpha-1 chain (IgA). Each of these spots has a similar molecular weight, but a different isoelectric point due to glycosylation. Similarly, 5 isoelectric isoforms of prothrombin, 3 isoelectric isoforms of complement factor H, two isoelectric isoforms of heparin cofactor II, and three molecular weight isoforms of haptoglobin were identified. Thus, while 38 unique protein isoforms displayed differential expression, this represented 17 unique protein identifications.

Each of the 38 protein spots, either alone or in combination, may be useful diagnostic markers to discriminate between the 5 clinical disease groups. To determine the combination of proteins likely to give the best diagnostic indicator, K-nearest neighbour discriminant analysis was performed using the DeCyder Extended Data Analysis module. From these proteins, discriminatory analysis identified 18 proteins (ranked 1-10, with some ranks having multiple proteins) that could together distinguish between the 5 clinical groups with 90% accuracy.

**Example 2 - Differential protein expression between healthy controls, asthmatics and COPD patients, irrespective of ex-smoking status**

Whilst ex-smoking status has some influence on the plasma protein profiles, the inventors next sought to combine the clinical groups to discount smoking status. Thus groups 1 and 2 were combined as all healthy controls, and groups 3 and 4 combined as all stable asthmatics. This analysis identified 72 proteins displaying significantly altered expression by 1-ANOVA, of which 57 were positively identified by MS (Table 2). As expected, a number of these are the same proteins as those found to be altered across all 5 disease groups.

Table 2 - Differentially expressed proteins between the 3 disease groups (all healthy controls; all asthmatics; COPD)

| Master spot no. | ANOVA p-value | Rank | Protein ID | Accession # | Differential Expression Ratio (*p<0.05, t-test) | | |
|---|---|---|---|---|---|---|---|
| | | | | | Asthmatics / Controls | COPD / Controls | COPD / Asthmatics |
| *Immunoglobulins* | | | | | | | |
| 1001 | 0.0019 | 1 | IgA | P01876 | 1.45* | 2.38* | 1.64* |
| 992 | 0.0055 | | IgA | P01876 | 1.19 | 1.98* | 1.66* |
| 1009 | 0.0084 | | IgA | P01876 | 1.24 | 1.85* | 1.49* |
| 1005 | 0.016 | | IgA | P01876 | 1.33 | 2.11* | 1.58 |
| 1017 | 0.017 | | IgA | P01876 | 1.2 | 1.94* | 1.62* |
| 1018 | 0.022 | | IgA | P01876 | 1.11 | 1.86* | 1.67* |
| 1022 | 0.022 | | IgA | P01876 | 1.16 | 1.86* | 1.61* |
| 996 | 0.024 | | IgA | P01876 | -1.36 | 1.3 | 1.77* |
| 1011 | 0.034 | | IgA | P01876 | 1.22 | 1.92* | 1.57 |
| 1010 | 0.039 | | IgA | P01876 | 1.3 | 1.99* | 1.53 |
| 806 | 0.011 | | IgM heavy chain | P01871 | -2.17* | -2.04 | 1.07 |
| 796 | 0.02 | | IgM heavy chain | P01871 | -1.8* | -2.68* | -1.49 |
| 794 | 0.026 | | IgM heavy chain | P01871 | -2.09* | -2.2 | -1.06 |
| 811 | 0.018 | | IgM heavy chain | P01871 | -1.74* | -2.22* | -1.27 |
| 804 | 0.04 | | IgM heavy chain | P01871 | -1.87* | -2.42 | -1.3 |
| *Coagulation cascade* | | | | | | | |
| 822 | 0.04 | 2 | Prothrombin | P00734 | -1.08 | -1.24* | -1.15 |
| 824 | 0.0054 | | Prothrombin | P00734 | -1.1 | -1.31* | -1.19 |
| 818 | 0.016 | | Prothrombin | P00734 | -1.14* | -1.26* | -1.1 |
| 819 | 0.018 | | Prothrombin | P00734 | -1.13* | -1.19* | -1.05 |
| 1242 | 0.0019 | | Fibrinogen gamma | P02679 | 1.06 | 1.31* | 1.24* |
| 1235 | 0.03 | | Fibrinogen gamma | P02679 | 1.18 | 1.48* | 1.26 |
| 1153 | 0.011 | | Fibrinogen gamma | P02679 | 1.25* | 1.28* | 1.02 |
| 859 | 0.0041 | | Heparin cofactor II | P05546 | 1.18* | -1.25 | -1.47* |
| 865 | 0.0097 | | Heparin cofactor II | P05546 | 1.1 | -1.2* | -1.32* |
| 611 | 0.037 | | Heparin cofactor II | P05546 | 1.31* | 1.05 | -1.24 |
| 911 | 0.0028 | | Protein S precursor | P07225 | -1.28* | -1.19 | 1.07 |
| 1152 | 0.0084 | | Fibrin beta | | 1.2* | 1.51* | 1.26 |

(continued)

**Coagulation cascade**

| No. | Protein | UniProt | Ratio 1 | Ratio 2 | Ratio 3 | p-value |
|---|---|---|---|---|---|---|
| 559 | ITI heavy chain H4 | Q14624 | 1.08 | -1.15 | -1.24* | 0.025 |
| 557 | ITI heavy chain H4 | Q14624 | -1.09 | -1.25* | -1.14* | 0.031 |
| 558 | ITI heavy chain H4 | Q14624 | -1.13 | -1.29* | -1.14 | 0.049 |
| 1284 | alpha-2-antiplasmin | P08697 | 1.07 | 1.27* | 1.19 | 0.036 |
| 967 | alpha-2 antiplasmin | P08697 | 1.75* | 2.07* | 1.18 | 0.0085 |
| 857 | Histidine-rich glycoprotein | P04196 | -1.3* | -1.22 | 1.07 | 0.041 |
| 1116 | Antithrombin-III | P01008 | 1.08 | -1.15 | -1.23* | 0.048 |
| 1106 | Antithrombin-III | P01008 | -1.04 | -1.22* | -1.18* | 0.019 |
| 737 | Gelsolin | P06396 | -1.21 | -1.43* | -1.19 | 0.042 |
| 236 | Alpha-2 macroglobulin | P01023 | NA | NA | -1.31* | 0.0034 |
| 495 | Alpha-2-macroglobulin | P01023 | 1.2 | -1.02 | -1.23* | 0.022 |
| 498 | Alpha-2 macroglobulin | P01023 | 1.14 | -1.17 | -1.34* | 0.032 |
| 249 | Alpha-2-macroglobulin | P01023 | NA | NA | -1.3* | 0.041 |

**Complement Pathways**

| No. | Protein | UniProt | Ratio 1 | Ratio 2 | Ratio 3 | p-value |
|---|---|---|---|---|---|---|
| 349 | 3 Complement factor H | P08603 | 1.03 | 1.34 | 1.3* | 0.028 |
| 338 | Complement factor H | P08603 | 1.82* | 1.19 | -1.53* | 0.00017 |
| 336 | Complement factor H | P08603 | 1.97* | 1.59* | -1.24* | 0.0031 |
| 340 | Complement factor H | P08603 | 1.66* | 1.27 | -1.3* | 0.0045 |
| 339 | Complement factor H | P08603 | 1.56* | 1.81* | 1.16 | 0.0078 |
| 680 | Complement factor B | P00751 | -1.2 | -1.5* | -1.25 | 0.015 |
| 688 | Complement factor B | P00751 | -1.34* | -1.2 | 1.12 | 0.029 |
| 547 | Complement 3 | P01024 | -1.49 | -1.76* | -1.18 | 0.032 |
| 691 | Complement component C4-A | P0C0L4 | -1.23* | -1.59* | -1.29* | 0.0034 |

**Iron Metabolism**

| No. | Protein | UniProt | Ratio 1 | Ratio 2 | Ratio 3 | p-value |
|---|---|---|---|---|---|---|
| 480 | Ceruloplasmin (ferroxidase) | P00450 | -1.35* | -1.34* | 1.01 | 0.002 |
| 1437 | Haptoglobin | P00738 | -1.31 | 1.48 | 1.94* | 0.0066 |
| 1394 | Haptoglobin | P00738 | -1.34 | 1.59 | 2.13* | 0.016 |
| 1444 | Haptoglobin | P00738 | -1.33 | 1.37 | 1.83* | 0.022 |
| 1403 | Haptoglobin | P00738 | -1.18 | 1.59 | 1.88* | 0.027 |
| 1381 | Haptoglobin | P00738 | -1.55 | 1.37 | 2.13* | 0.03 |

(continued)

**_Iron Metabolism_**

| | | | | | | |
|---|---|---|---|---|---|---|
| 968 | 0.024 | Hemopexin | <u>P02790</u> | -1.2* | NA | NA |
| 965 | 0.029 | Hemopexin | <u>P02790</u> | -1.12 | -1.23* | -1.1 |
| 977 | 0.033 | Hemopexin | <u>P02790</u> | -1.34* | -1.2 | -1.12 |

The proteins were analysed by K-nearest neighbour discriminant analysis in the EDA module. Discriminatory analysis identified three proteins that together gave 100% accuracy for differentiating between the three clinical groups (Table 2; Rank 1, 2, 3). The relative expression ratio of each of these spots (Table 2) shows that spot 1001, IgA, is increased in asthmatics compared to healthy controls, and is further increased in COPD patients. Spot 822, prothrombin, is decreased in COPD patients compared to healthy controls. Finally spot 349, complement factor H, is increased in asthmatics and trends towards an increase in COPD patients (1.34 fold, p=0.10) compared to healthy controls.

The DIGE discriminatory analysis suggests that the combined expression of IgA, prothrombin and complement factor H can effectively distinguish between the three clinical groups. To confirm this a META analysis was performed on these three proteins. The combined result for the three proteins together is statistically significant and stronger than the individual proteins alone, and with a smaller variance. Thus, as predicted by the EDA analysis, combining the expression changes of the 3 proteins together gives a more powerful diagnostic than any of the individual proteins alone.

**Example 3 - Confirmation of discriminatory proteins**

As described above in Example 2, the combined expression levels of IgA, prothrombin and complement factor H in plasma can discriminate between healthy controls, stable asthmatics, and COPD patients. To confirm the 2D-DIGE analysis, and to move towards a cheaper diagnostic tool for routine clinical use, the inventors determined the relative expression of each of these proteins using either ELISA or immunoblotting. Both the ELISA and immunoblotting analysis detects the total protein expression of all isoforms in the serum, whilst the 2D-DIGE analyses each individual isoform separately. Thus while 2D-DIGE analysis of IgA spot 1001 showed increased expression in asthmatics, and more so in COPD patients, 9 other IgA protein spots are significantly increased in COPD patients but not in asthmatics (Table 4). In agreement with this, the ELISA analysis of total IgA showed a significant increase in the COPD patients only, compared to both healthy controls and asthmatics (data not shown). Prothrombin displayed decreased expression in COPD patients compared to healthy controls. In addition, 3 other prothrombin isoforms (spots 818, 819 and 824; Table 4) were identified, with slightly different isoelectric points, and each of these displayed a significant reduction in the COPD patients. Immunoblotting for total prothrombin confirmed these results, with a 1.4 (p=0.008) and 1.3 (p=0.014) fold reduction in the COPD group compared to healthy controls and asthmatics respectively. By 2D-DIGE complement factor H was significantly increased in asthmatics, and showed a trend (1.34 fold; p=0.10) to increased expression in COPD compared to healthy controls. When total complement factor H protein levels were assessed by immunoblotting, only a slight, yet significant, increase was observed in the asthmatic group compared to healthy controls (1.13 fold, p=0.013). A far greater increase is observed in the COPD patients (1.7 fold, p<0.0001 and 1.5 fold, p<0.0001) compared to healthy controls and asthmatics respectively.

To determine the discriminatory power of the ELISA and immunoblotting data for these proteins, meta-analysis was performed. Similarly to the meta-analysis for the 2D-DIGE protein spots, the expression values of the combined three proteins is statistically significant and stronger than the individual proteins alone, and with a smaller variance (data not shown).

**Part 2 - Clinical evaluation of biomarkers**

Validation of the diagnostic value of biomarkers in clinical populations represents a crucial step in the development of a commercially viable diagnostic. The clinical design strategy used at this clinical stage (Part 2) differs from the strategy adopted in the Part 1 study described above. In Part 1 samples were collected from distinct patient groups and many different proteins screened for their discriminatory potential. In the clinical phase the biomarkers were tested in a much larger number of patients, and in several different patient groups. This involved patients who are representative of the clinical spectrum of patients in whom the tests will be used, examination of the optimal cut-point (test threshold) in patient groups, assessment of the test in patient groups with varying disease prevalence, and assessment of the biomarkers in diseases that may alter biomarker levels but are unrelated to its intended use.

In this clinical validation the inventors recruited and investigated a large group of subjects with asthma, COPD, and mixed obstructive airway diseases. This was also a group of older patients (aged >55years) where it is accepted that diagnosis of airways disease is difficult using traditional methods. 76 older subjects in this group were sampled and characterized. Patients were classified as healthy control, asthma, or COPD. In addition, a new clinical group, mixed obstructive airway disease was evaluated, where patients have features of both asthma and COPD. The clinical characterisation of patients in this clinical trial is summarised in Table 3.

Table 3. Clinical patient characterisations

| | Healthy Controls | Stable Asthma | COPD | Overlap Asthma-COPD | P value |
|---|---|---|---|---|---|
| Number of patients | 23 | 14 | 22 | 14 | |
| Age (Years)[a] | 49.9 ± 17.6 | *67.7 ± 6.7 | *68.0 ± 7.9 | *68.7 ± 9.0 | <0.0001 |
| Sex (Male/ Female)[c] | 11/12 | 8/6 | 8/14 | 5/9 | 0.566 |
| Smoking, n(%)[c]: | | | | | 0.759 |
| Never | 12 (52.2%) | 7 (50%) | 8 (36.4%) | 5 (35.7%) | |
| Ex | 11 (47.8%) | 7 (50%) | 13 (59.1%) | 8 (57.1%) | |
| Current | 0 | 0 | 1 (4.6%) | 1 (7.1%) | |
| Pack years[a] | 13.9 ± 15.6 | 39.5 ± 28.6 | 39.9 ± 36.9 | 37.2 ± 37.9 | 0.151 |
| Atopy, n(%)[c] | 6(26.1%) | *11 (78.6%) | 8 (36.4%) | 9 (64.3%) | 0.006 |
| %predicted $FEV_1$[a] | 109.0 ± 13.8 | *61.6 ± 23.8 | *60.6 ± 22.7 | *55.0 ±17.1 | <0.0001 |
| %predicted FVC[a] | 111.0 ± 14.3 | *79.7 ± 21.0 | *81.9 ± 22.2 | *81.2 ± 15.8 | <0.0001 |
| $FEV_1$/FVC %[a] | 80.7 ± 6.7 | *60.8 ± 16.2 | *57.7 ± 14.1 | *53.4 ± 14.2 | <0.0001 |
| $PD_{15}$ (mL) [b, d] | N/A | 7.4 (3.5,19.6) | 5.9 (4.9, 8.7) | 6.3 (2.9,11.2) | 0.935 |
| $D_{LCO}$[a] | N/A | 73.3 ± 17.3 | 72.5 ± 23.2 | 84.1 ± 21.2 | 0.323 |
| ICS use, n(%)[c] | 0 | 11 (78.6%) | 21 (95.5%) | 14 (100%) | 0.08 |
| ICS (µg beclomethasone equivalents /day)[b] | N/A | 1600 ± 780 | 1460 ± 646 | 1657 ± 511 | 0.659 |
| Sputum Cell Counts | | | | | |
| Quality [b] | 18 (16, 19) | 17 (16, 19) | 18.5 (17.5,19) | 17.5(16,18) | 0.250 |
| Total cell count x $10^6$/ml [b] | 1.7 (1.3,3.1) | 4.0 (2.8, 8.5) | 3.3 (1.9,7.6) | 3.7 (1.4,9.5) | 0.039 |
| Neutrophils% [b] | 28.5 (13.3, 54.5) | 62.9 (45.5, 78.5) | *56.0 (35.5, 88.3) | *77.0 (45.8, 85.5) | 0.003 |
| Eosinophils% [b] | 0.25 (0, 0.5) | *1.65 (0.5, 3.25) | *2.0 (0.75, 7.25) | *1.0 (0.25, 5.5) | 0.0001 |
| Macrophages% [b] | 63.8 (41.5, 77.8) | 29.9 (16.8, 52.0) | *15.0 (8.0,44.0) | *17.5 (7.3,37.3) | 0.0003 |
| Lymphocytes% [b] | 0.75 (0.25, 1.75) | 0 (0, 0.5) | 0.25 (0, 1.0) | 0.25 (0, 1.0) | 0.062 |
| Columnar epithelial% [b] | 1.0 (0.25,7.5) | 1.5 (0.5, 2.0) | 0.75 (0.25, 2.25) | 1.0 (0.5,2.25) | 0.659 |
| Squamous% [b] | 4.5 (1.96,8.3) | 1.6 (0.7,18.4) | 2.7 (0.99, 8.9) | 3.9 (2.2, 8.5) | 0.725 |

[a]Values are Mean ± SD, oneway ANOVA; [b]Values are median (interquartile range), Kruskall-Wallis test; [c]Chi Square or Fisher's exact test; [d] $PD_{15}$ is provocation dose resulting in 15% drop in baseline $FEV_1$ expressed as geometric mean (log SD); $FEV_1$ is forced expiratory volume in 1 second; FVC is forced vital capacity; $D_{LCO}$ is carbon monoxide diffusing capacity; ICS is inhaled corticosteroids. Bonferroni post hoc test significant compared to: *Healthy Controls

Serum samples were collected from a panel of elderly individuals and analysed for serum levels of the candidate proteins identified in Part 1, as well as additional biomarkers. The candidate markers tested were haptoglobin, α-2-macroglobulin, ceruloplasmin, hemopexin, complement 3 and C4a, antithrombin III using ELISA assays developed previously. The levels of prothrombin, Complement Factor B and Complement Factor H were tested using western blot at the clinical trial level.

## Example 4 - Individual biomarkers in diagnosis of obstructive airways diseases

All clinical samples were investigated for expression of individual biomarkers and the results were analysed as disease versus healthy control and disease versus disease groups. Differential expression profiles are summarised in Table 4. Serum samples from patients with asthma, COPD and mixed disease showed significant up-regulation of haptoglobin, ceruloplasmin, and hemopexin expressions in comparison with the control group. Expression of α-2-macroglobulin showed significant differences between asthma and COPD groups, and mixed disease and asthma groups. This up-regulation of α-2-macroglobulin related to COPD disease status, indicating that α-2-macroglobulin is a differential marker specific for chronic obstructive airway diseases. Haptoglobin, ceruloplasmin and α-2-macroglobulin showed diagnostic consistency between the preclinical study (Part 1; n=58) and the clinical study (Part 2; n=76).

Table 4. Clinical validation of individual biomarker in disease diagnosis

| Marker | Differential Expression | | | | | |
| | Asthma v Control | COPD v Control | Mixed v Control | COPD v Asthma | Mixed v Asthma | Mixed v COPD |
|---|---|---|---|---|---|---|
| Haptoglobin | ↑** | ↑* | ↑** | × | × | × |
| α-2-macroglobulin | × | ↑* | × | ↑* | ↑* | × |
| Ceruloplasmin | ↑*** | ↑* | × | × | × | × |
| Hemopexin | ↑*** | ↑** | ↑* | × | × | × |
| × no significant difference; ↑ upregulated; * 0.01 < p < 0.05; **0.001< p < 0.01; *** p < 0.001 | | | | | | |

## Example 5 - Biomarker combination in obstructive airways disease diagnosis

The difference in protein expression between the disease groups was statistically assessed as a composite score. Proteins of interest were combined in a meta-analysis for each of the comparisons:

asthma vs healthy; COPD vs healthy; mixed vs healthy; COPD vs asthma; mixed vs asthma; and mixed vs COPD.

Statistical analysis of the biomarker combinations was performed as for the previous studies. After assessing the results for these four selected markers, haptoglobin, hemopexin α-2-macroglobulin, and ceruloplasmin, a composite score using logistic regression was created. The power of all possible combinations of these proteins was also investigated. From these comparisons a quantitative summary estimate was derived for the discriminatory value of the diagnostic biomarkers and marker suite. The results are summarised below.

### Asthma vs control

Table 5. Asthma vs Control logistic regression (n=36):

| | Coefficient (95%CI) | P value | Model p value |
|---|---|---|---|
| **Individual protein** | | | |
| Ceruloplasmin | 0.009 (0.002, 0.015) | 0.008 | **0.0007** |
| Haptoglobin | 0.501 (0.10, 0.90) | 0.014 | **0.002** |
| Hemopexin | 0.019 (0.006, 0.31) | 0.004 | **0.0002** |
| α-2-macroglobulin | -0.196 (-1.12, 0.73) | 0.677 | 0.672 |

(continued)

| Combination proteins | | | |
|---|---|---|---|
| Ceruloplasmin | 0.009 (0.001, 0.016) | 0.022 | **0.0002** |
| Haptoglobin | 0.434 (0.008, 0.86) | 0.046 | |
| Ceruloplasmin | 0.005 (-0.0005, 0.01) | 0.074 | **0.0001** |
| Hemopexin | 0.015 (0.002, 0.029) | 0.024 | |
| | 1.25 (0.25, 2.25) | 0.014 | **<0.00001** |
| Haptoglobin | 0.027 (0.005, 0.049) | 0.015 | |
| Hemopexin | | | |

Table 6 summarises the statistical analysis for cut-off predicted values to exclude and include asthma respectively.

Table 6. Asthma vs Control: Cut off and sensitivity /specificity to rule out disease

| | Cut off measurement | Cut-off, Predicted value | Sensitivity (95%CI) | Specificity (95%CI) | LR+ | LR - |
|---|---|---|---|---|---|---|
| Ceruloplasmin | >=744.93 mcg/ml | >=0.293 | 85.71 | 68.18 | 2.69 | 0.21 |
| Haptoglobin | >=6.36 mg/ml | >=0.313 | 85.71 | 54.55 | 1.89 | 0.26 |
| Hemopexin | >=402.35 mg/ml | >=0.162 | 100.00 | 50.0 | 2.0 | 0 |
| $\alpha$-2-macroglobulin | <=2.89 mg/ml | >=0.386 | 64.29 | 65.22 | 1.84 | 0.55 |
| Haptoglobin / Ceruloplasmin | | >=0.342 | 85.71 | 72.73 | 3.14 | 0.20 |
| Hemopexin / Ceruloplasmin | | >=0.226 | 92.31 | 68.18 | 2.91 | 0.113 |
| Hemopexin / Haptoglobin | | >=0.289 | 100.0 | 90.91 | 11.0 | 0 |
| ***Asthma vs Control: Cut off and sensitivity/specificity to rule in disease*** | | | | | | |
| | Cut off measurement | Cut-off, Predicted value | Sensitivity (95%CI) | Specificity (95%CI) | LR + | LR - |
| Ceruloplasmin | >=898.31 mcg/ml | >=0.607 | 50.0 | 95.45 | 11.0 | 0.52 |
| Haptoglobin | >=8.70 mg/ml | >=0.596 | 50.0 | 86.36 | 3.67 | 0.58 |
| Hemopexin | >=479.65 mg/ml | >=0.453 | 69.23 | 86.36 | 5.08 | 0.36 |
| $\alpha$-2-macroglobulin | <=2.85 mg/ml | >=0.388 | 57.14 | 69.57 | 1.88 | 0.62 |
| Haptoglobin / Ceruloplasmin | | >=0.561 | 64.29 | 95.45 | 14.14 | 0.37 |
| Hemopexin / Ceruloplasmin | | >=0.421 | 69.23 | 81.82 | 3.81 | 0.38 |
| Hemopexin / Haptoglobin | | >=0.457 | 92.31 | 95.45 | 20.31 | 0.08 |

The results above demonstrate that ceruloplasmin, haptoglobin and hemopexin can discriminate between asthma and healthy controls, and any combinations of these three markers increases their discriminatory power in asthma diagnosis. The combination of haptoglobin and hemopexin showed significantly high sensitivity and specificity in disease diagnosis.

***COPD vs control***

Table 7. COPD vs Control logistic regression (n=44):

|  | *Coefficient (95%Cl)* |  | *P value* | *Model p value* |
|---|---|---|---|---|
| **Individual protein** |  |  |  |  |
| Ceruloplasmin | 0.005 (0.0004, 0.0089) |  | 0.032 | **0.014** |
| Haptoglobin | 0.246 (0.003, 0.489) |  | 0.047 | **0.034** |
| Hemopexin | 0.009 (0.002, 0.017) |  | 0.019 | **0.009** |
| $\alpha$-2-macroqlobulin | 0.69 (0.04, 1.35) |  | 0.038 | **0.016** |
| **Combination proteins** |  |  |  |  |
| Ceruloplasmin | 0.004 (-0.0005, 0.008) |  | 0.079 | **0.016** |
| Haptoglobin | 0.185 (-0.067, 0.44) |  | 0.150 |  |
| Ceruloplasmin Hemopexin | 0.003 (-0.0008, 0.008) | 0.007 (-0.0006, 0.016) | 0.110 0.071 | **0.008** |
|  | 0.167 (-0.089, 0.423) |  | 0.200 | **0.014** |
| *Haptoglobin* | 0.008 (-0.0004, 0.016) |  | 0.061 |  |
| *Hemopexin* |  |  |  |  |
| $\alpha$-2-macroglobulin | 0.618 (-0.097, 1.33) |  | 0.090 | **0.006** |
| Hemopexin | 0.009 (0.0004, 0.016) |  | 0.039 |  |

Table 8. COPD vs Control: Cut off and sensitivity /specificity to rule out disease

|  | *Cut off measurement* | *Cut-off, Predicted value* | *Sensitivity (95%Cl)* | *Specificity (95%Cl)* | *LR+* | *LR -* |
|---|---|---|---|---|---|---|
| Ceruloplasmin | >=692.70 mcg/ml | >=0.414 | 81.82 | 50.0 | 1.63 | 0.36 |
| Haptoglobin | >=5.17 mg/ml | >=0.422 | 77.27 | 40.91 | 1.31 | 0.56 |
| Hemopexin | >=462.65 mg/ml | >=0.523 | 72.73 | 86.36 | 5.33 | 0.32 |
| $\alpha$-2-macroglobulin | >=3.29 mg/ml | >=0.456 | 72.73 | 69.57 | 2.39 | 0.39 |
| Haptoglobin / Ceruloplasmin |  | >=0.460 | 72.73 | 59.09 | 1.78 | 0.46 |
| *Hemopexin / Ceruloplasmin* |  | >=0.521 | 72.73 | 77.27 | 3.2 | 0.35 |
| *Hemopexin / Haptoglobin* |  | >=0.439 | 72.73 | 63.64 | 2.0 | 0.43 |
| *$\alpha$-2-macroglobulin / Hemopexin* |  | >=0.412 | 86.36 | 68.18 | 2.71 | 0.20 |
| **COPD vs Control: Cut off and sensitivity /specificity to rule in disease** |  |  |  |  |  |  |
|  | *Cut off measurement* | *Cut-off Predicted value* | *Sensitivity (95%Cl)* | *Specificity (95%Cl)* | *LR* | *LR -* |
| Ceruloplasmin | >=800.27 mcg/ml | >=0.537 | 54.55 | 72.73 | 2. | 0.63 |
| Haptoglobin | >=7.81 mg/ml | >=0.582 | 50.0 | 81.82 | 2. 5 | 0.61 |

(continued)

**COPD vs Control: Cut off and sensitivity /specificity to rule in disease**

| | Cut off measurement | Cut-off Predicted value | Sensitivity (95%CI) | Specificity (95%CI) | LR | LR - |
|---|---|---|---|---|---|---|
| Hemopexin | >=517.64 mg/ml | >=0.652 | 50.0 | 95.45 | 11 | 0.52 |
| $\alpha$-2-macroglobulin | >=3.79 mg/ml | >=0.544 | 50.0 | 91.3 | 5. 5 | 0.55 |
| Haptoglobin / Ceruloplasmin | | >=0.573 | 63.64 | 81.82 | 3.5 | 0.44 |
| *Hemopexin / Ceruloplasmin* | | >=0.617 | 63.64 | 90.91 | 7.0 | 0.40 |
| *Hemopexin / Haptoglobin* | | >=0.567 | 59.09 | 95.45 | 13.0 | 0.43 |
| *$\alpha$-2-macroglobulin / Hemopexin* | | >=0.631 | 54.55 | 95.45 | 12.0 | 0.47 |

As shown above, all four markers, ceruloplasmin, haptoglobin, hemopexin and $\alpha$-2-macroglobulin, can discriminate between COPD and healthy controls. Combinations of these markers increase their discriminatory power in COPD diagnosis.

### Mixed (*asthma and COPD*) vs control

Table 9. Mixed vs Control logistic regression (n=36)

| | Coefficient (95%CI) | P value | Model p value |
|---|---|---|---|
| **Individual protein** | | | |
| Ceruloplasmin | 0.004 (-0.001,0.008) | 0.119 | 0.092 |
| Haptoglobin | 0.659 (0.16, 1.15) | 0.009 | **0.0005** |
| Hemopexin | 0.018 (0.004,0.033) | 0.010 | **0.002** |
| $\alpha$-2-macroglobulin | 0.73 (-0.11, 1.56) | 0.089 | 0.071 |
| **Combination proteins** | | | |
| Ceruloplasmin | 0.002 (-0.004, 0.009) | 0.468 | 0.002 |
| Haptoglobin | 0.63 (0.12, 1.14) | 0.016 | |
| Ceruloplasmin | 0.002 (-0.004, 0.007) | 0.552 | **0.007** |
| Hemopexin | 0.017 (0.002, 0.032) | 0.022 | |
| | 0.01 (-0.007, 0.008) | 0.866 | **0.0007** |
| *Ceruloplasmin* | 0.02 0.016 (-0.001, 0.03) | 0.069 | |
| | 0.03 0.588 (0.056, 1.12) | 0.030 | |
| *Hemopexin* | | | |
| *Haptoglobin* | | | |
| $\alpha$-2-macroglobulin | 1.12 (-0.24, 2.47) | 0.106 | **0.0002** |
| Hemopexin | 0.015 (-0.002, 0.033) | 0.078 | |
| Haptoglobin | 0.64 (0.066, 1.21) | 0.029 | |

Table 10. Mixed vs Control: Cut off and sensitivity /specificity to rule out disease

| | Cut off measurement | Cut-off, Predicted value | Sensitivity (95%CI) | Specificity (95%CI) | LR+ | LR - |
|---|---|---|---|---|---|---|
| Ceruloplasmin | >=694.91 mcg/ml | >=0.341 | 71.43 | 50.0 | 1.43 | 0.57 |
| Haptoglobin | >=6.64 mg/ml | >=0.318 | 85.71 | 68.18 | 2.69 | 0.21 |
| Hemopexin | >=435.08 mg/ml | >=0.335 | 85.71 | 63.64 | 2.36 | 0.22 |
| α-2-macroqlobulin | >=3.07 mg/ml | >=0.330 | 71.43 | 56.52 | 1.64 | 0.51 |
| Haptoglobin / Ceruloplasmin | | >=0.321 | 92.86 | 59.09 | 2.27 | 0.12 |
| *Hemopexin / Ceruloplasmin* | | >=0.321 | 92.86 | 68.18 | 2.92 | 0.10 |
| *Hemopexin / Haptoglobin / Ceruloplasmin* | | >=0.277 | 92.86 | 59.09 | 2.27 | 1.12 |
| Haptoglobin / α-2-macroglobulin / Hemopexin | | >=0.295 | 100.0 | 77.27 | 4.40 | 0 |

**Mixed vs Control: Cut off and sensitivity /specificity to rule in disease**

| | Cut off measurement | Cut-off, Predicted value | Sensitivity (95%CI) | Specificity (95%CI) | LR+ | LR - |
|---|---|---|---|---|---|---|
| Ceruloplasmin | >=785.22 mcg/ml | >=0.420 | 50.0 | 72.73 | 1.83 | 0.69 |
| Haptoglobin | >=7.74 mg/ml | >=0.490 | 71.43 | 81.82 | 3.93 | 0.35 |
| Hemopexin | >=454.53 mg/ml | >=0.420 | 64.29 | 81.82 | 3.54 | 0.44 |
| α-2-macroglobulin | >=3.50 mg/ml | >=0.401 | 50.0 | 82.61 | 2.88 | 0.61 |
| Haptoglobin / Ceruloplasmin | | >=0.488 | 64.29 | 81.82 | 3.54 | 0.44 |
| *Hemopexin / Ceruloplasmin* | | >=0.464 | 64.29 | 86.36 | 4.71 | 0.41 |
| *Hemopexin / Haptoglobin / Ceruloplasmin* | | >=0.530 | 71.43 | 95.45 | 15.71 | 0.30 |
| *Haptoglobin / α-2-macroglobulin / Hemopexin* | | >=0.435 | 92.86 | 95.45 | 20.43 | 0.07 |

As shown above haptoglobin and hemopexin showed good diagnostic value individually in mixed disease diagnosis and each of these markers in combination with ceruloplasmin extended their diagnostic values in disease diagnosis. Both haptoglobin and hemopexin in combination with either ceruloplasmin or α-2-macroglobulin showed significantly increased potential in disease diagnosis with the combination of haptoglobin, hemopexin and α-2-macroglobulin showed powerful diagnostic value with great sensitivity and specificity.

***COPD vs asthma***

Table 11. COPD vs Asthma logistic regression (n=36):

| | Coefficient (95%CI) | P value | Model p value |
|---|---|---|---|
| **Individual protein** | | | |
| Ceruloplasmin | -0.001 (-0.005, 0.002) | 0.414 | 0.408 |
| Haptoglobin | -0.14 (-0.41, 0.14) | 0.323 | 0.310 |

(continued)

| | Coefficient (95%CI) | P value | Model p value |
|---|---|---|---|
| **Individual protein** | | | |
| Hemopexin | -0.003 (-0.01, 0.004) | 0.346 | 0.334 |
| $\alpha$-2-macroglobulin | 0.94 (0.051, 1.82) | **0.038** | **0.013** |
| **Combination proteins** | | | |
| $\alpha$-2-macroglobulin | 1.28 (0.17, 2.39) | **0.024** | **0.008** |
| Haptoglobin | -0.30(-0.66, 0.05) | 0.088 | |
| $\alpha$-2-macroglobulin | 1.21 (0.14, 2.28) | **0.026** | **0.012** |
| Ceruloplasmin | -0.003 (-0.007, 0.001) | 0.118 | |
| $\alpha$-2-macroglobulin | 1.89 (0.31, 3.49) | **0.019** | **0.003** |
| Haptoglobin | -0.59 (-1.13, -0.04) | **0.034** | |
| Hemopexin | -0.002 (-0.01, 0.008) | 0.749 | |
| $\alpha$-2-macroglobulin | 1.44 (0.24, 2.64) | **0.019** | **0.011** |
| Haptoglobin | -0.27 (-0.65, 0.10) | 0.153 | |
| Ceruloplasmin | -0.003 (-0.007, 0.002) | 0.234 | |

Table 12. COPD vs Asthma: Cut off and sensitivity /specificity to rule out disease

| | Cut off measurement | Cut-off, Predicted value | Sensitivity (95%CI) | | Specificity (95%CI) | LR+ | LR - |
|---|---|---|---|---|---|---|---|
| $\alpha$-2-macroglobulin | >=3.29 mg/ml | >=0.585 | 72.73 | | 71.43 | 2.546 | 0.382 |
| $\alpha$-2-macroglobulin Haptoglobin | | >=0.556 | 72.73 | | 64.29 | 2.036 | 0.424 |
| $\alpha$-2-macroglobulin Ceruloplasmin | | >=0.585 | 72.73 | 71.43 | 2.546 | | 0.382 |
| $\alpha$-2-macroglobulin Haptoglobin Hemopexin | | >=0.512 | 81.82 | 61.54 | 2.009 | | 0.369 |
| $\alpha$-2-macroglobulin Haptoglobin Ceruloplasmin | | >=0.520 | 86.36 | 64.29 | 2.418 | | 0.212 |

| COPD vs Asthma: Cut off and sensitivity /specificity to rule in disease | | | | | | |
|---|---|---|---|---|---|---|
| | Cut off measurement | Cut-off, Predicted value | Sensitivity (95%CI) | Specificity (95%CI) | LR+ | LR - |
| $\alpha$-2-macroglobulin | >=3.68 mg/ml | >=0.672 | 54.55 | 92.86 | 7.636 | 0.4 90 |
| $\alpha$-2-macroglobulin Haptoglobin | | >=0.621 | 68.18 | 92.86 | 9.545 | 0.39 2 |
| $\alpha$-2-macroglobulin Ceruloplasmin | | >=0.665 | 68.18 | 92.86 | 9.545 | 0.343 |
| $\alpha$-2-macroglobulin Haptoglobin Hemopexin | | >=0.686 | 68.18 | 100.0 | | 0.409 |

As shown above, $\alpha$-2-macroglobulin alone can distinguish asthma from COPD with great statistical confidence. In combination with haptoglobin, hemopexin and ceruloplasmin, the diagnostic power of $\alpha$-2-macroglobulin in differen-

tiating asthma from COPD is increased.

## Example 6 - Prevalence of co-morbid conditions in the secondary validation population

Five medical conditions were identified as potential confounders of the blood based marker diagnosis panel: hepatobiliary disease, ischemic coronary disease, obesity, endocrine and metabolic disorders, and psychiatric conditions. Table 13 summarises a co-morbidity analysis of the secondary validation population and indicates no significant effect of these co-morbid conditions on the marker levels, apart from an effect of psychiatric disease on levels of $\alpha$-2 macroglobulin.

Table 13. Biomarker levels by co-morbidities

| Biomarker | No Diabetes N= 66 | Diabetes N=6 | P value |
|---|---|---|---|
| Ceruloplasmin, mcg/ml[b] | 785(668, 906) | 717 (695, 749) | 0.328 |
| Haptoglobin, mg/ml[a] | 7.0 ± 2.6 | 8.3 ± 3.0 | 0.255 |
| Hemopexin, mcg/ml[a] | 469 ± 95.5 | 493 ± 39.6 | 0.549 |
| $\alpha$-2-macroglobulin, mg/ml[b] | 3.2(2.8, 3.8) | 3.8(2.4, 4.1) | 0.962 |
| | BMI<=30 N=54 | BMI>30 N=19 | |
| Ceruloplasmin mcg/ml[b] | 800 (668, 898) | 732 (680, 875) | 0.754 |
| Haptoglobin, mg/ml[a] | 6.9 ± 2.8 | 7.6 ± 2.1 | 0.354 |
| Hemopexin, mcg/ml[a] | 470 ± 94 | 476 ± 90 | 0.806 |
| $\alpha$-2-macroglobulin, mg/ml[b] | 3.2 (2.8, 3.7) | 3.0 (2.7, 4.1) | 0.880 |
| | No Cardiac disease N=60 | Cardiac disease n=12 | |
| Ceruloplasmin, mcg/ml[b] | 793 (666, 910) | 721 (681, 783) | 0.257 |
| Haptoglobin, mg/ml[a] | 6.9 ± 2.7 | 8.1 ± 2.3 | 0.158 |
| Hemopexin, mcg/ml[a] | 474 ± 95 | 460 ± 83 | 0.632 |
| $\alpha$-2-macroglobulin, mg/ml[b] | 3.1 (2.8, 3.7) | 3.7 (3.2, 4.1) | 0.181 |
| | No Liver disease N=67 | Liver disease N=5 | |
| Ceruloplasmin, mcg/ml[b] | 766 (668, 898) | 800 (708, 801) | 0.938 |
| Haptoglobin, mg/ml[a] | 7.2 ± 2.7 | 7.0 ± 3.0 | 0.867 |
| Hemopexin, mcg/ml[a] | 477 ± 85 | 400 ± 158 | 0.072 |
| $\alpha$-2-macroglobulin, mg/ml[b] | 3.2 (2.8, 3.8) | 3.7 (2.7, 5.0) | 0.600 |
| | No Psychiatric disorder, n=55 | Psychiatric disorder, n=17 | |
| Ceruloplasmin, mcg/ml[b] | 785 (680, 913) | 708 (652, 820) | 0.210 |
| Haptoglobin, mg/ml[a] | 7.2 ± 2.5 | 6.9 ± 3.2 | 0.626 |
| Hemopexin, mcg/ml[a] | 464 ± 88 | 493 ± 103 | 0.262 |
| $\alpha$-2-macroglobulin, mg/ml[b] | 3.1 (2.7, 3.6) | 3.7 (3.5, 4.5) | 0.031 |

[a]Values are Mean ± SD, Student's tests; [b]Values are median (interquartile range), Wicoxon rank sum test.

## Example 7 - Biomarker analysis in sputum

The proteomic markers that were identified as diagnostic for obstructive airway disease were then assayed in sputum supernatant and results compared to serum analysis. Each of the blood biomarkers was present in airway samples from patients with asthma and COPD. Both haptoglobin and ceruloplasmin were found to have diagnostic discrimination when assayed from induced sputum as individual markers (Table 14).

Table 14. Induced sputum proteomic biomarkers in asthma, COPD and healthy controls

| Disease | Hemopexin | Haptoglobin | $\alpha$-2-macroglobulin | Ceruloplasmin |
|---|---|---|---|---|
| **Blood** | | | | |
| HC | 1085 ± 189 μg/mL | 2.72 ± 0.97 mg/mL | 2676 ± 766 μg/mL | 735 ± 174 μg/mL |
| A | 1091 ± 156 μg/mL | 4.32 ± 1.44 mg/mL | 2343 ± 807 μg/mL | 1049 ± 278 μg/mL |
| COPD | 973 ± 148 μg/mL | 3.58 ± 0.84 mg/mL | 2829 ± 587 μg/mL | 906 ± 187 μg/mL |
| **Sputum** | | | | |
| HC | 733 ± 457 ng/mL | 304 ± 266 ng/mL | 1.1 ± 1.7 μg/mL | 27.7 ± 9.4 μg/mL |
| A | 781 ± 512 ng/mL | **837 ± 912 ng/mL \*** | 1.9 ± 3.5 μg/mL | 33.1 ± 17.4 μg/mL |
| COPD | 883 ± 674 ng/mL | **802 ± 668 ng/mL \*** | 2.3 ± 1.5 μg/mL | **50.4 ± 28.3 μg/mL \*** |
| Mean ± SD. <br> \*p<0.05; HC healthy control; A asthma; COPD: chronic obstructive pulmonary disease. | | | | |

**Example 8 - Biomarkers to distinguish asthma subtypes**

Using proteomic technologies as described above the present inventors have also evaluated the proteins listed in Table 2 (see Example 2) for their ability to discriminate between eosinophilic, neutrophilic and paucigranulocytic asthma.

Plasma samples from 26 well characterised asthmatics (classified as having eosinophilic, neutrophilic, or paucigranulocytic asthma) were immuno-depleted and the proteins were separated on 2D-gels. Airway inflammatory phenotype was established by induced sputum examination of these subjects. The 2D-gel images from plasma samples were analysed to identify potential markers of inflammatory phenotype. The relative abundance of each protein spot was compared between inflammatory phenotypes using methodologies as described above. A range of protein markers was identified which were differentially expressed between the phenotypes. To further evaluate these biomarkers identified for their clinical diagnostic potential, a range of these potential biomarkers from the highly ranked candidates from proteomic analysis was selected for a pre-clinical validation trial.

Serum from asthmatic individuals was analysed for candidate marker concentration. The ability of each biomarker to discriminate between the inflammatory phenotypes was assessed individually and in combination. A realistic and powerful targeted approach is the development of combinatorial biomarkers: biomarker patterns that typically consist of a few individual parameters. Combinatorial biomarker patterns confer significantly more information than a single measurement. Logistic regression was applied to assess the combination of multiple biomarkers instead of single marker analysis and a method was developed to combine the diagnostic value of multiple biomarkers, which provides more comprehensive power for sensitivity and specificity analysis with great confidence. The discriminatory potential of these biomarkers for inflammatory phenotype diagnosis was analysed by utilising quantitative ELISA method.

To validate the markers selected from the initial marker identification process, a large group of asthmatic subjects was recruited for an expanded cross-sectional study. This was a group of patients with average age between 52-67 years, where the diagnosis of asthma using traditional methods is accepted. The inventors sampled and characterized 71 asthmatics and 15 healthy subjects in this clinical trial (n=86). Patients were classified as eosinophilic asthma, neutrophilic asthma, or paucigranulocytic asthma sufferers. Serum samples were collected from patients in this clinical trial, and the candidate markers identified were validated by using quantitative ELISA. Table 15 provides the mean expression levels of individual biomarkers in comparisons between inflammatory phenotypes as measured by ELISA (standard deviation in parentheses). Table 16 provides the statistical analysis of this expression data indicating the combinations of markers that are statistically significant in terms of their ability to discriminate between phenotypes. This data demonstrates that serum markers, either alone or in combination, can identify and discriminate asthma inflammatory phenotypes. For discrimination between eosinophilic and neutrophilic asthma expression of antithrombin 3 and complement 3 are increased in eosinophilic asthma when compared to neutrophilic asthma, whereas expression of comp,ement factor B, haptoglobin and hemopexin are decreased in eosinophilic asthma when compared to neutrophilic asthma. For discrimination between paucigranulocytic and non-paucigranulocytic asthma expression of antithrombin 3 is increased in paucigranulocytic asthma when compared to non-paucigranulocytic asthma whereas expression of complement 4a is decreased in paucigranulocytic asthma when compared to non-paucigranulocytic asthma. For discrimination between eosinophilic and paucigranulocytic asthma expression of complement 4a is increased in eosinophilic asthma when compared to paucigranulocytic asthma, whereas expression of complement factor B, haptoglobin, antithrombin 3 and complement 4a are decreased in eosinophilic asthma when compared to paucigranulocytic asthma. For discrimination between neutrophilic and paucigranulocytic asthma expression of complement 4a is increased in neutrophilic asthma when compared to paucigranulocytic asthma, whereas expression of antithrombin 3, complement 3 and ceruloplasmin are decreased in neutrophilic asthma when compared to paucigranulocytic asthma.

Table 15. Mean expression levels of markers in asthma inflammatory phenotypes

| Marker[1] | Paucigranulocytic | Non Paucigranulocytic |
|---|---|---|
| At3 | 1.11 (0.53) | 0.84 (0.47) |
| C4a, mcg/ml | 26.84 (15.07) | 36.49 (12.29) |
| | **Eosinophilic** | **Neutrophilic** |
| At3 | 0.85 (0.48) | 0.63 (0.19) |
| C3 | 2.10(0.64) | 1.73 (0.55) |
| CFB | 110.88 (86.27) | 230.98 (319.18) |
| Haptoglobin | 1.52 (0.70) | 1.84 (0.55) |
| Hemopexin | 1.39 (0.27) | 1.51 (0.33) |
| | **Eosinophilic** | **Paucigranulocytic** |
| At3 | 0.85 (0.48) | 1.11 (0.53) |
| C4a, mcg/ml | 37.22 (12.37) | 26.84 (15.07) |
| CFB | 110.88 (86.27) | 158.17 (128.18) |
| Haptoglobin | 1.52 (0.70) | 1.89 (0.91) |
| | **Neutrophilic** | **Paucigranulocytic** |
| At3 | 0.63 (0.19) | 1.11 (0.53) |
| C3 | 1.73 (0.55) | 2.03 (0.37) |
| C4a, mcg/ml | 36.20 (8.07) | 26.84 (15.07) |
| Ceruloplasmin | 464.31 (69.61) | 519.55(97.27) |
| [1]At3, antithrombin3; C4a, complement 4a; C3, complement 3; CFB, complement factor B. | | |

Table 16. Statistical analysis of biomarkers for asthma inflammatory phenotypes

| Asthma subtype comparison | Marker/ Marker Combination | Logistic regression | | | |
|---|---|---|---|---|---|
| | | Constant | Coefficient | p-value | Model p-value |
| Paucigranulocytic v Non paucigranulocytic (n=71) | | | | | |
| | C4a<br>Antithrombin 3 | -0.4332 | -0.0552<br>1.0176 | 0.020<br>0.085 | 0.008 |
| Eosinophilic v Neutrophilic (n=44) | | | | | |
| | Antithrombin 3 | -0.8362 | 1.6635 | 0.096 | 0.063 |
| | C3 | -1.6294 | 1.0446 | 0.057 | 0.046 |
| | CFB | 1.3100 | -0.0067 | 0.087 | 0.026 |
| | Haptoglobin | 1.7257 | -0.8077 | 0.117 | 0.102 |
| | Hemopexin | 2.3852 | -1.3955 | 0.196 | 0.186 |
| | C3<br>Haptoglobin | -0.2237 | 1.2786<br>-1.0982 | 0.034<br>0.064 | 0.019 |
| | Antithrombin 3<br>C3 | -2.6445 | 3.3173<br>1.8560 | 0.002<br>0.011 | 0.0008 |

(continued)

| Eosinophilic v Neutrophilic (n=44) | | | | | |
|---|---|---|---|---|---|
| | Haptoglobin | | -1.7468 | 0.018 | |
| | Antithrombin 3 | -1.4951 | 2.5907 | 0.044 | 0.009 |
| | C3 | | 1.4256 | 0.022 | |
| | Hemopexin | | -1.8914 | 0.135 | |
| | C3 | 0.3112 | 1.3150 | 0.035 | 0.013 |
| | CFB | | -0.0046 | 0.248 | |
| | Haptoglobin | | -0.6735 | 0.301 | |
| | Antithrombin 3 | -2.3965 | 3.3360 | 0.023 | 0.002 |
| | C3 | | 1.8630 | 0.011 | |
| | Haptoglobin | | -1.6814 | 0.043 | |
| | | | -0.2648 | 0.868 | |
| | Antithrombin 3 | 0.8026 | 2.070 | 0.074 | 0.044 |
| | Complement Factor B | | -0.0048 | 0.271 | |
| | Haptoglobin | | -0.8652 | 0.249 | |
| | Hemopexin | | 0.1233 | 0.935 | |
| | Antithrombin 3 | 0.1177 | 1.6249 | 0.125 | 0.019 |
| | Complement Factor B | | -0.0067 | 0.091 | |
| | Antithrombin 3 | 0.7025 | 2.2300 | 0.046 | 0.017 |
| | Haptoglobin | | -1.1679 | 0.047 | |
| | Antithrombin 3 | 1.1330 | 1.6927 | 0.120 | 0.038 |
| | Complement Factor B | | -0.0054 | 0.211 | |
| | Hemopexin | | -0.8525 | 0.490 | |
| Eosinophilic v Paucigranulocytic (n=42) | | | | | |
| | Antithrombin 3 | 1.5029 | -1.0463 | 0.108 | 0.099 |
| | C4a | -1.3897 | 0.0580 | 0.031 | 0.018 |
| | CFB | 1.0612 | -0.0044 | 0.167 | 0.156 |
| | Haptoglobin | 1.5214 | -0.6103 | 0.150 | 0.138 |
| | Antithrombin 3 | -0.3912 | -1.4529 | 0.080 | 0.010 |
| | C4a | | 0.0691 | 0.025 | |
| | C4a | -1.2876 | 0.0908 | 0.011 | 0.003 |
| | CFB | | -0.0093 | 0.027 | |
| | Antithrombin 3 | -0.4712 | -1.3138 | 0.141 | 0.003 |
| | C4a | | 0.1012 | 0.009 | |
| | CFB | | -0.0086 | 0.043 | |
| | C4a | -0.5565 | 0.0936 | 0.014 | 0.006 |
| | CFB | | -0.0089 | 0.035 | |
| | | | -0.5270 | 0.310 | |
| | Antithrombin 3 | -0.1011 | -1.1859 | 0.197 | 0.006 |
| | C4a | | 0.1040 | 0.012 | |
| | CFB | | -0.0086 | 0.045 | |
| | Haptoglobin | | -0.3662 | 0.505 | |
| Neutrophilic v Paucigranulocytic (n=34) | | | | | |
| | Antithrombin 3 | 3.1194 | -3.6995 | 0.013 | 0.0006 |

(continued)

| Neutrophilic v Paucigranulocytic (n=34) | | | | | | |
|---|---|---|---|---|---|---|
| | C3 | 2.7504 | -1.3963 | 0.083 | 0.065 | |
| | C4a | -2.1895 | 0.0723 | 0.046 | 0.023 | |
| | Ceruloplasmin | 4.365 | -0.0086 | 0.077 | 0.052 | |
| | Antithrombin 3 C4a | -0.0593 | -6.8767 0.1744 | 0.007 0.018 | <0.0001 | |
| | Antithrombin 3 C3 C4a | 7.3666 | -11.1065 -3.6040 0.2506 | 0.011 0.040 0.021 | <0.0001 | |
| | Antithrombin 3 C3 Ceruolplasmin | 0.5490 | -6.5813 0.1734 -0.0016 | 0.824 | 0.0001 | |
| | Antithrombin 3 C3 C4a Cerulopasmin | 10.0739 | -10.5749 -3.8827 0.2593 -0.0055 | 0.021 0.045 0.028 0.521 | <0.0001 | |

Using the data from Table 16 the following logistic regression equation can be used to determine the predictive value of mean marker expression levels in a given subject in diagnosing an asthma inflammatory phenotype or subtype.

$$\ln(p/(1-p)) = \beta_0 + \beta_i x_i$$

$$p = \exp(\beta_0 + \beta_i x_i)/(1 + \exp(\beta_0 + \beta_i x_i))$$

**References**

Anderson, L. (2005). Candidate-based proteomics in the search for biomarkers of cardiovascular disease. J Physiol 563, 23-60.

Johns DP, Crockett AJ. (2005). Lung Function testing, In Evidence based Respiratory Medicine Gibson PG (ed.), Blackwell publishing Limited, Oxford UK.

Liu, T., Qian, W.J., Mottaz, H.M., Gritsenko, M.A., Norbeck, A.D., Moore, R.J., Purvine, S.O., Camp, D.G., 2nd, and Smith, R.D. (2006). Evaluation of multiprotein immunoaffinity subtraction for plasma proteomics and candidate biomarker discovery using mass spectrometry. Mol Cell Proteomics 5, 2167-2174.

Pinto-Plata, V., Toso, J., Lee, K., Park, D., Bilello, J., Mullerova, H., De Souza, M.M., Vessey, R., Celli, B. (2007) Profiling serum biomarkers in patients with COPD: associations with clinical parameters. Thorax 62,595-601.

Verrills, N.M., Liem, N.L., Liaw, T.Y., Hood, B.D., Lock, R.B., and Kavallaris, M. (2006). Proteomic analysis reveals a novel role for the actin cytoskeleton in vincristine resistant childhood leukemia - An in vivo study. Proteomics 6, 1681-1694.

Woof, J.M., and Kerr, M.A. (2006). The function of immunoglobulin A in immunity. J Pathol 208, 270-282.

**Claims**

1. A method for diagnosing an obstructive airways disease selected from asthma, chronic obstructive pulmonary disease (COPD) and mixed (asthma and COPD) disease in a subject, the method comprising determining the level of expression of ceruloplasmin and hemopexin, and optionally one or more proteins selected from haptoglobin and $\alpha$-2-macroglobulin, in an *in vitro* biological sample from the subject, wherein an elevation in the expression level of ceruloplasmin and hemopexin, and optionally one or more proteins selected from haptoglobin and $\alpha$-2-macroglobulin, in the *in vitro* biological sample from the subject compared to the expression level of ceruloplasmin and hemopexin, and optionally one or more proteins selected from haptoglobin and $\alpha$-**2-macroglobulin** from one or more control samples, is indicative of said obstructive airways disease.

2. The method of claim 1 wherein the sample is selected from whole blood, blood plasma, blood serum, sputum, saliva or urine.

3. The method of claim 1 or claim 2 wherein the control sample is derived from one or more individuals known not to suffer from an obstructive airways disease selected from asthma, COPD or mixed (asthma and COPD) disease, or from one or more individuals known to have asthma, COPD or mixed (asthma and COPD) disease.

4. The method of claim 3 wherein

   (i) when the control sample is derived from one or more individuals known to have asthma, the method enables the diagnosis of COPD in the subject; and
   (ii) when the control sample is derived from one or more individuals known to have COPD, the method enables the diagnosis of asthma in the subject.

5. The method of any one of claims 1 to 4 wherein increased expression of ceruloplasmin, hemopexin and haptoglobin in a sample from the subject relative to the expression of the corresponding proteins in one or more control samples from individual(s) known not to suffer from asthma is indicative that the subject suffers from asthma.

6. The method of any one of claims 1 to 4 wherein increased expression of ceruloplasmin, hemopexin, $\alpha$-**2-macroglobulin** and optionally haptoglobin in a sample from the subject relative to the expression of the corresponding proteins in one or more control samples from individual(s) known not to suffer from COPD is indicative that the subject suffers from COPD.

7. The method of any one of claims 1 to 4 wherein increased expression of ceruloplasmin hemopexin, $\alpha$-**2-macroglobulin** and optionally haptoglobin in a sample from the subject relative to the expression of the corresponding proteins in one or more control samples from individual(s) known to suffer from asthma is indicative that the subject suffers from mixed (asthma and COPD) disease.

8. The method according to claim 1 wherein when the subject is known to suffer from an obstructive airways disease selected from asthma or COPD, the method comprises further determining the expression level of $\alpha$-**2-macroglobulin** in an *in vitro* sample from the subject, wherein an elevation in the expression level of $\alpha$-**2-macroglobulin** in the sample compared to the expression level of $\alpha$-**2-macroglobulin** in one or more control samples derived from an individual known to have asthma, is indicative that the subject has COPD.

9. The method of any one of claims 1 to 4 wherein increased expression of ceruloplasmin and hemopexin, and optionally haptoglobin and $\alpha$-**2-macroglobulin** in a sample from the subject relative to the expression of the corresponding proteins in one or more control samples from individual(s) known not to suffer from asthma and COPD is indicative that the subject suffers from mixed (asthma and COPD) disease.

10. The method of any one of claims 1 to 9, further comprising determining the level of expression of one or more of the additional proteins identified in Table 2 herein.

11. A method for determining a treatment regime for a subject suffering from an obstructive airways disease selected from asthma, COPD and mixed (asthma a COPD) disease, the method comprising:

   (a) determining the level of ceruloplasmin and hemopexin, and optionally one or more proteins selected from haptoglobin and $\alpha$-2-macroglobulin, in an *in vitro* biological sample from the subject, optionally further comprising comparing the expression level of the proteins in the *in vitro* sample from the subject to the expression level of the corresponding proteins from control samples;

(b) diagnosing the obstructive airways disease on the basis of the determination in (a); and

(c) selecting an appropriate treatment regime for the subject on the basis of the diagnosis.

12. A method for determining disease control in a subject suffering from an obstructive airways disease selected from asthma, COPD and mixed (asthma and COPD) disease, the method comprising:

(a) determining the level of ceruloplasmin and hemopexin, and optionally one or more proteins selected from haptoglobin and α-**2-macroglobulin** in an *in vitro* biological sample from the subject; and

(b) repeating at least step (a) at least once over a period of time and determining whether the levels of the proteins change over the period of time and optionally,

(c) comparing the level of the proteins in the sample from the subject at one or more points in time to the expression profile of the corresponding proteins from one or more control samples,

wherein changes in the levels obtained in (b) and optionally (c) are indicative of disease control in the subject.

**Patentansprüche**

1. Verfahren zur Diagnose einer obstruktiven Atemwegserkrankung, ausgewählt aus Asthma, chronisch-obstruktiver Lungenerkrankung (COPD) und einer Mischerkrankung (Asthma und COPD), bei einem Probanden, wobei das Verfahren das Bestimmen des Expressionsniveaus von Ceruloplasmin und Hämopexin und gegebenenfalls einem oder mehreren Proteinen, ausgewählt aus Haptoglobin und α-2-Makroglobulin, in einer biologischen *In-vitro*-Probe von einem Probanden umfasst, wobei eine Erhöhung des Expressionsniveaus von Ceruloplasmin und Hämopexin und gegebenenfalls einem oder mehreren Proteinen, ausgewählt aus Haptoglobin und α-2-Makroglobulin, in der biologischen *In-vitro*-Probe von dem Probanden verglichen mit dem Expressionsniveau von Ceruloplasmin und Hämopexin und gegebenenfalls einem oder mehreren Proteinen, ausgewählt aus Haptoglobin und α-2-Makroglobulin, von einer oder mehreren Kontrollproben für diese obstruktive Atemwegserkrankung indikativ ist.

2. Verfahren nach Anspruch 1, wobei die Probe aus Vollblut, Blutplasma, Blutserum, Sputum, Speichel oder Urin ausgewählt ist.

3. Verfahren nach Anspruch 1 oder Anspruch 2, wobei die Kontrollprobe von einem oder mehreren Individuen, die bekanntermaßen nicht an einer obstruktiven Atemwegserkrankung, ausgewählt aus Asthma, COPD oder einer Mischerkrankung (Asthma und COPD), leiden, oder von einem oder mehreren Individuen, die bekanntermaßen Asthma, COPD oder eine Mischerkrankung (Asthma und COPD) haben, stammt.

4. Verfahren nach Anspruch 3, wobei

(i) wenn die Kontrollprobe von einem oder mehreren Individuen stammt, die bekanntermaßen Asthma haben, das Verfahren die Diagnose von COPD bei dem Probanden ermöglicht; und

(ii) wenn die Kontrollprobe von einem oder mehreren Individuen stammt, die bekanntermaßen COPD haben, das Verfahren die Diagnose von Asthma bei dem Probanden ermöglicht.

5. Verfahren nach einem der Ansprüche 1 bis 4, wobei eine erhöhte Expression von Cerulo-plasmin, Hämopexin und Haptoglobin in einer Probe von dem Probanden in Bezug auf die Expression der entsprechenden Proteine in einer oder mehreren Kontrollproben von dem/den Individuum/Individuen, die bekanntermaßen nicht an Asthma leiden, indikativ dafür ist, dass der Proband an Asthma leidet.

6. Verfahren nach einem der Ansprüche 1 bis 4, wobei eine erhöhte Expression von Cerulo-plasmin, Hämopexin, α-2-Makroglobulin und gegebenenfalls Haptoglobin in einer Probe von dem Probanden in Bezug auf die Expression der entsprechenden Proteine in einer oder mehreren Kontrollproben von dem/den Individuum/Individuen, die bekanntermaßen nicht an COPD leiden, indikativ dafür ist, dass der Proband an COPD leidet.

7. Verfahren nach einem der Ansprüche 1 bis 4, wobei eine erhöhte Expression von Cerulo-plasmin, Hämopexin, α-2-Makroglobulin und gegebenenfalls Haptoglobin in einer Probe von dem Probanden in Bezug auf die Expression der entsprechenden Proteine in einer oder mehreren Kontrollproben von dem/den Individuum/Individuen, die bekanntermaßen an Asthma leiden, indikativ dafür ist, dass der Proband an einer Mischerkrankung (Asthma und COPD) leidet.

8. Verfahren nach Anspruch 1, wobei, wenn der Proband bekanntermaßen an einer obstruktiven Atemwegserkrankung, ausgewählt aus Asthma oder COPD, leidet, das Verfahren ferner das Bestimmen des Expressionsniveaus von $\alpha$-2-Makroglobulin in einer *In-vitro-Probe* von dem Proband umfasst, wobei eine Erhöhung des Expressionsniveaus von $\alpha$-2-Makroglobulin in der Probe verglichen mit dem Expressionsniveau von $\alpha$-2-Makroglobulin in einer oder mehreren Kontrollproben, die von einem Individuum stammen, das bekanntermaßen Asthma hat, indikativ dafür ist, dass der Proband COPD hat.

9. Verfahren nach einem der Ansprüche 1 bis 4, wobei eine erhöhte Expression von Ceruloplasmin und Hämopexin und gegebenenfalls Haptoglobin und $\alpha$-2-Makroglobulin in einer Probe von dem Proband in Bezug auf die Expression der entsprechenden Proteine in einer oder mehreren Kontrollproben von dem/den Individuum/Individuen, die bekanntermaßen nicht an Asthma und COPD leiden, indikativ dafür ist, dass der Proband an einer Mischer-krankung (Asthma und COPD) leidet.

10. Verfahren nach einem der Ansprüche 1 bis 9, ferner umfassend das Bestimmen des Expressionsniveaus von einem oder mehreren der zusätzlichen Proteine, die in Tabelle 2 identifiziert sind.

11. Verfahren zur Bestimmung eines Behandlungsregimes für einen Probanden, der an einer obstruktiven Atemwegserkrankung, ausgewählt aus Asthma, COPD und einer Mischerkrankung (Asthma und COPD), leidet, wobei das Verfahren:

(a) das Bestimmen des Niveaus an Ceruloplasmin und Hämopexin und gegebenenfalls von einem oder mehreren Proteinen, ausgewählt aus Haptoglobin und $\alpha$-2-Makroglobulin, in einer biologischen *In-vitro-Probe* von dem Probanden, gegebenenfalls ferner umfassend das Vergleichen des Expressionsniveaus der Proteine in der *In-vitro*-Probe von dem Probanden mit dem Expressionsniveau der entsprechenden Proteine aus den Kontrollproben;
(b) das Diagnostizieren der obstruktiven Atemwegserkrankung auf der Basis der Bestimmung in (a) und
(c) das Auswählen eines geeigneten Behandlungsregimes für den Probanden auf der Basis der Diagnose umfasst.

12. Verfahren zur Bestimmung der Krankheitskontrolle bei einem Probanden, der an einer obstruktiven Atemwegserkrankung, ausgewählt aus Asthma, COPD und einer Mischerkrankung (Asthma und COPD), leidet, wobei das Verfahren:

(a) das Bestimmen des Niveaus an Ceruloplasmin und Hämopexin und gegebenenfalls von einem oder mehreren Proteinen, ausgewählt aus Haptoglobin und $\alpha$-2-Makroglobulin, in einer biologischen In-*vitro*-Probe von dem Probanden und
(b) das zumindest einmalige Wiederholen von zumindest Schritt (a) über einen Zeitraum und das Bestimmen, ob sich die Niveaus der Proteine über den Zeitraum verändern, und gegebenenfalls
(c) das Vergleichen des Niveaus der Proteine in der Probe von dem Probanden zu einem oder mehreren Zeitpunkten mit dem Expressionsprofil der entsprechenden Proteine von einer oder mehreren Kontrollproben umfasst,

wobei Veränderungen der in (b) und gegebenenfalls (c) erhaltenen Niveaus für die Krankheitskontrolle bei dem Probanden indikativ sind.

## Revendications

1. Procédé de diagnostic d'une maladie obstructive des voies respiratoires choisie parmi l'asthme et une bronchopneumopathie chronique obstructive (BPCO) et une maladie mixte (asthme et BPCO) chez un sujet, le procédé comprenant la détermination du niveau d'expression de la céruloplasmine et de l'hémopexine, et facultativement d'une ou plusieurs protéines choisies parmi l'haptoglobine et l'$\alpha$-2-macroglobuline, dans un échantillon biologique in vitro du sujet, dans lequel une élévation dans le niveau d'expression de la céruloplasmine et de l'hémopexine, et facultativement d'une ou plusieurs protéines choisies parmi l'haptoglobine et l'$\alpha$-2-macroglobuline, dans l'échantillon biologique in vitro du sujet comparé au niveau d'expression de la céruloplasmine et de l'hémopexine, et facultativement d'une ou plusieurs protéines choisies parmi l'haptoglobine et l'$\alpha$-2-macroglobuline d'un ou plusieurs échantillons témoins, est indicatif de ladite maladie obstructive des voies respiratoires.

2. Procédé selon la revendication 1, dans lequel l'échantillon est choisi parmi le sang entier, le plasma sanguin, le sérum sanguin, le crachat, la salive ou l'urine.

3. Procédé selon la revendication 1 ou la revendication 2, dans lequel l'échantillon témoin est dérivé d'un ou plusieurs individus dont on sait qu'ils ne souffrent pas d'une maladie obstructive des voies respiratoires choisie parmi l'asthme, la BPCO ou une maladie mixte (asthme et BPCO), ou d'un ou plusieurs individus dont on sait qu'ils souffrent d'asthme, de BPCO, et d'une maladie mixte (asthme et BPCO).

4. Procédé selon la revendication 3, dans lequel

(i) lorsque l'échantillon témoin est dérivé d'un ou plusieurs individus dont on sait qu'ils souffrent d'asthme, le procédé permet le diagnostic de la BPCO chez le sujet ; et
(ii) lorsque l'échantillon témoin est dérivé d'un ou plusieurs individus dont on sait qu'ils souffrent de BPCO, le procédé permet le diagnostic de l'asthme chez le sujet.

5. Procédé selon l'une quelconque des revendications 1 à 4, dans lequel une expression accrue de la céruloplasmine, de l'hémopexine et de l'haptoglobine dans un échantillon du sujet par rapport à l'expression des protéines correspondantes dans un ou plusieurs échantillons témoins d'un ou plusieurs individu(s) dont on sait qu'il(s) ne souffre(nt) pas d'asthme est indicatif que le sujet souffre d'asthme.

6. Procédé selon l'une quelconque des revendications 1 à 4, dans lequel une expression accrue de la céruloplasmine, de l'hémopexine, de l'$\alpha$-2-macroglobuline et facultativement de l'haptoglobine dans un échantillon d'un sujet par rapport à l'expression des protéines correspondantes dans un ou plusieurs échantillons témoins d'un un ou plusieurs échantillons témoins d'un ou plusieurs individu(s) dont on sait qu'il(s) ne souffre(nt) pas de BPCO est indicatif que le sujet souffre de BPCO.

7. Procédé selon l'une quelconque des revendications 1 à 4, dans lequel une expression accrue de la céruloplasmine, de l'hémopexine, de l'$\alpha$-2-macroglobuline et facultativement de l'haptoglobine dans un échantillon du sujet par rapport à l'expression des protéines correspondantes dans un ou plusieurs échantillons témoins d'un un ou plusieurs échantillons témoins d'un ou plusieurs individu(s) dont on sait qu'il(s) souffre(nt) d'asthme est indicatif que le sujet souffre d'une maladie mixte (asthme et BPCO).

8. Procédé selon la revendication 1, dans lequel lorsque l'on sait que le sujet souffre d'une maladie obstructive des voies respiratoires choisie parmi l'asthme ou la BPCO, le procédé comprend en outre la détermination du niveau d'expression de l'$\alpha$-2-macroglobuline dans un échantillon in vitro d'un sujet, dans lequel une élévation du niveau d'expression de l'$\alpha$-2-macroglobuline dans l'échantillon comparé au niveau d'expression de l'$\alpha$-2-macroglobuline dans un ou plusieurs échantillons dérivés d'un individu dont on sait qu'il souffre d'asthme, est indicatif que le sujet souffre de BPCO.

9. Procédé selon l'une quelconque des revendications 1 à 4, dans lequel une expression accrue de la céruloplasmine, de l'hémopexine, et facultativement de l'haptoglobine et de l'$\alpha$-2-macroglobuline dans un échantillon du sujet par rapport à l'expression des protéines correspondantes dans un ou plusieurs échantillons témoins d'un ou plusieurs individu(s) dont on sait qu'il(s) souffre(nt) d'asthme et de BPCO est indicatif que le sujet souffre d'une maladie mixte (asthme et BPCO).

10. Procédé selon l'une quelconque des revendications 1 à 9, comprenant en outre la détermination du niveau d'expression d'une ou plusieurs des protéines supplémentaires identifiées dans le présent tableau II.

11. Procédé de détermination d'un régime de traitement pour un sujet souffrant d'une maladie obstructive des voies respiratoires choisie parmi l'asthme, la BPCO et une maladie mixte (asthme et BPCO), le procédé comprenant :

(a) la détermination du niveau de la céruloplasmine et de l'hémopexine, et facultativement d'une ou plusieurs protéines choisies parmi l'haptoglobine et l'$\alpha$-2-macroglobuline, dans un échantillon biologique in vitro du sujet, comprenant facultativement en outre la comparaison du niveau d'expression des protéines dans l'échantillon in vitro du sujet au niveau d'expression des protéines correspondantes d'échantillons témoins ;

(b) le diagnostic de la maladie obstructive des voies respiratoires sur la base de la détermination en (a) ; et
(c) la sélection d'un régime de traitement approprié pour le sujet sur la base du diagnostic.

**12.** Procédé de détermination de lutte contre la maladie chez un sujet souffrant d'une maladie obstructive des voies respiratoires choisie parmi l'asthme, la BPCO et une maladie mixte (asthme et BPCO), le procédé comprenant :

(a) la détermination du niveau de la céruloplasmine et de l'hémopexine, et facultativement d'une ou plusieurs protéines choisies parmi l'haptoglobine et l'$\alpha$-2-macroglobuline, dans un échantillon biologique in vitro du sujet ; et
(b) la répétition au moins de l'étape (a) au moins une fois sur une période de temps et le fait de déterminer si les niveaux des protéines changent sur la période de temps et facultativement,
(c) la comparaison du niveau des protéines dans l'échantillon du sujet à un ou plusieurs points dans le temps au profil d'expression des protéines correspondantes d'un ou plusieurs échantillons témoins,

dans lequel des changements dans les niveaux obtenus en (b) et facultativement (c) sont indicatifs d'une lutte contre la maladie chez le sujet.

## REFERENCES CITED IN THE DESCRIPTION

### Patent documents cited in the description

- WO 2008109730 A1
- WO 2006105252 A2

### Non-patent literature cited in the description

- **GREEN et al.** *Lancet,* 2002, vol. 360, 1715-1721
- **LERU et al.** *Rom J Intern Med.,* 1998, vol. 36 (1-2), 105-11
- **VYSOTNYUK et al.** *Likars'ka sprava,* 2006, (1-2), 65-81
- **SCHOONBROOD et al.** *Am J Respir Crit Care Med.,* 1994, vol. 150 (6), 1519-1527
- **SCHOONBROOD et al.** *Clin Chim Acta,* 1995, vol. 240 (2), 163-78
- **ENGSTRÖM et al.** *THORAX,* 2009, vol. 64, 211-215
- **SANDFORD et al.** *Eur Respir J,* 1997, vol. 10, 1380-1391
- **BAKER et al.** *Respiration,* 2000, vol. 67, 533-538
- **VAN RENSEN et al.** *Am J Respir Crit Care Med,* 2002, vol. 165, 1275-1279
- **ZAK-NEJMARK et al.** *Archivum Immunologiae et Therapiae Experimentalis,* 1986, vol. 34, 547-552
- **SYROMYATNIKOVA et al.** *Therapeutic Archives,* 1980, vol. 52 (7), 36-37
- **ANDERSON, L.** Candidate-based proteomics in the search for biomarkers of cardiovascular disease. *J Physiol,* 2005, vol. 563, 23-60
- Lung Function testing. **JOHNS DP ; CROCKETT AJ.** Evidence based Respiratory Medicine. Blackwell publishing Limited, 2005
- **LIU, T. ; QIAN, W.J. ; MOTTAZ, H.M. ; GRIT-SENKO, M.A. ; NORBECK, A.D. ; MOORE, R.J. ; PURVINE, S.O. ; CAMP, D.G. ; SMITH, R.D.** Evaluation of multiprotein immunoaffinity subtraction for plasma proteomics and candidate biomarker discovery using mass spectrometry. *Mol Cell Proteomics,* 2006, vol. 5, 2167-2174
- **PINTO-PLATA, V. ; TOSO, J. ; LEE, K. ; PARK, D. ; BILELLO, J. ; MULLEROVA, H. ; DE SOUZA, M.M. ; VESSEY, R. ; CELLI, B.** Profiling serum biomarkers in patients with COPD: associations with clinical parameters. *Thorax,* 2007, vol. 62, 595-601
- **VERRILLS, N.M. ; LIEM, N.L. ; LIAW, T.Y. ; HOOD, B.D. ; LOCK, R.B. ; KAVALLARIS, M.** Proteomic analysis reveals a novel role for the actin cytoskeleton in vincristine resistant childhood leukemia - An in vivo study. *Proteomics,* 2006, vol. 6, 1681-1694
- **WOOF, J.M. ; KERR, M.A.** The function of immunoglobulin A in immunity. *J Pathol,* 2006, vol. 208, 270-282